# EUROPEAN PATENT SPECIFICATION

(11) **EP 3 038 538 B1**
(45) Date of publication and mention of the grant of the patent: **14.11.2018**
(21) Application number: 14812891.1
(22) Date of filing: 19.06.2014
(51) Int. Cl.: A61B 17/00, A61B 17/04, A61B 17/34, A61B 17/06

(54) **APPARATUS FOR FASCIAL CLOSURE DEVICE FOR LAPAROSCOPIC TROCAR PORT SITE AND SURGERY**
VORRICHTUNG FÜR FASZIENVERSCHLUSSVORRICHTUNG FÜR PORTSTELLE EINES TROKARS FÜR DIE LAPAROSKOPIE UND CHIRURGIE
APPAREIL POUR DISPOSITIF DE FERMETURE FASCIALE POUR SITE D'ORIFICE DE TROCART LAPAROSCOPIQUE ET CHIRURGICAL

(30) Priority: 19.06.2013 US 201361837114 P; 03.09.2013 US 201361873325 P
(43) Date of publication of application: 06.07.2016
(73) Proprietor: Endolutions, LLC, Austin, TX 78759 (US)
(72) Inventor: PATEL, Manoj, B., Lumberton, NJ 08048 (US); ZHAO, Philip, Fort Lee, NJ 07024 (US); PATEL, Neal, North Brunswick, NJ 08902 (US); GILKEY, Landon, Austin, TX 78744 (US); ALBALA, David, M., Manlius, NY 13104 (US); CASTRO, Salvatore, Raleigh, NC 27603 (US)
(74) Representative: Gee, Steven William
(86) International application number: PCT/US2014/043274
(87) International publication number: WO 2014/205279

(56) References cited:
- EP-A1- 2 412 317
- WO-A1-02/24078
- US-A- 6 059 800
- US-A1- 2002 045 908
- US-A1- 2009 062 852
- US-A1- 2012 035 623
- US-B2- 7 060 077
- US-B2- 7 824 419

## Description

### BACKGROUND- FIELD OF INVENTION

This invention generally relates to laparoscopic surgery and trocar port placement. More particularly, the invention relates to tissue closure devices, including surgical suturing devices as well as such devices that can be used for intra-abdominal suturing and suturing of puncture wounds generated by surgical laparoscopic trocar ports and other puncturing devices.

### BACKGROUND- PRIOR ART

Apparatus and methods are provided for treating tissue opening, for example an endoscopic trocar port opening used in a minimally invasive surgical procedure. A suture placement device is provided to rapidly, safely, efficiently and effectively close tissue defects created to access the intra-abdominal cavity during laparoscopic surgical procedures in a human body. The device as described is able to obtain adequate tissue adjacent to the tissue defect to provide a strong closure, to maintain pneumoperitoneum needed for appropriate visualization of the peritoneal contents during the closure process, and to protect the vital structures within the abdominal cavity in the vicinity and the healthcare provider for risk of injury.

The prior art discloses various methods of routing and presenting a suture so that suture ends can be tied to close an opening in tissue after a cannular device is removed from the tissue.

Example prior art devices include US Patent No. 7,060,077 to Gordon et al., US Patent Publication No. 2012/0035623 to Bagaoisan et al., US Patent Publication No. 2008/0033459 Shafi et al., US Patent No. 8,109,943 to Boraiah et al., the Neat Stitch™ closure device, and US Patent Publication No. 2013/0165956 to Sherts et al. EP 2 412317 A1 discloses a suturing device including an elongate shaft and a guide movably mounted to the elongate shaft. A guard member is mounted on the shaft, the guard member having at least one arm which is movable from a first retracted position in which it is substantially aligned with the elongate shaft and a second deployed position. A suture is releasably mounted to an attachment member of the guard member. The guide has an angled bore which can be aligned with the attachment member.

### TAP block

It is desirable in endoscopic and robotic surgery to perform a TAP block (transverse abdominal plane) and/or to inject an anti-inflammatory agent in proximity to a trocar port in order to provide short term or prolonged post-operative pain relief for a patient.

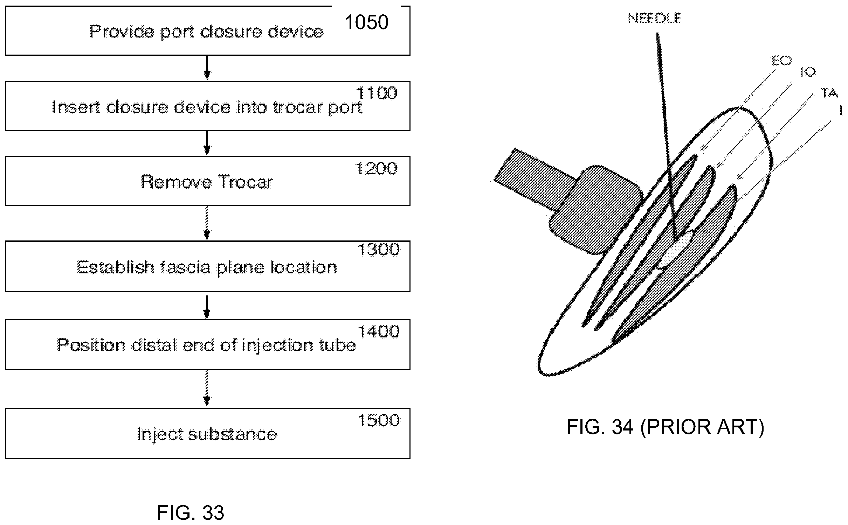

FIG. 34 is a prior art illustration of a TAP block. In this method, an ultrasound probe is used to determine when a needle tip is in a desired position between internal oblique muscle (IO) and the transversus abdominis (TA) so that a local anasthetic (I) may be injected. This intramuscular region between the internal oblique muscle (IO) and the transversus abdominis (TA) is also the desired injection site for steroids or NSAIDs (non-steroidal anti-inflammatory drugs).

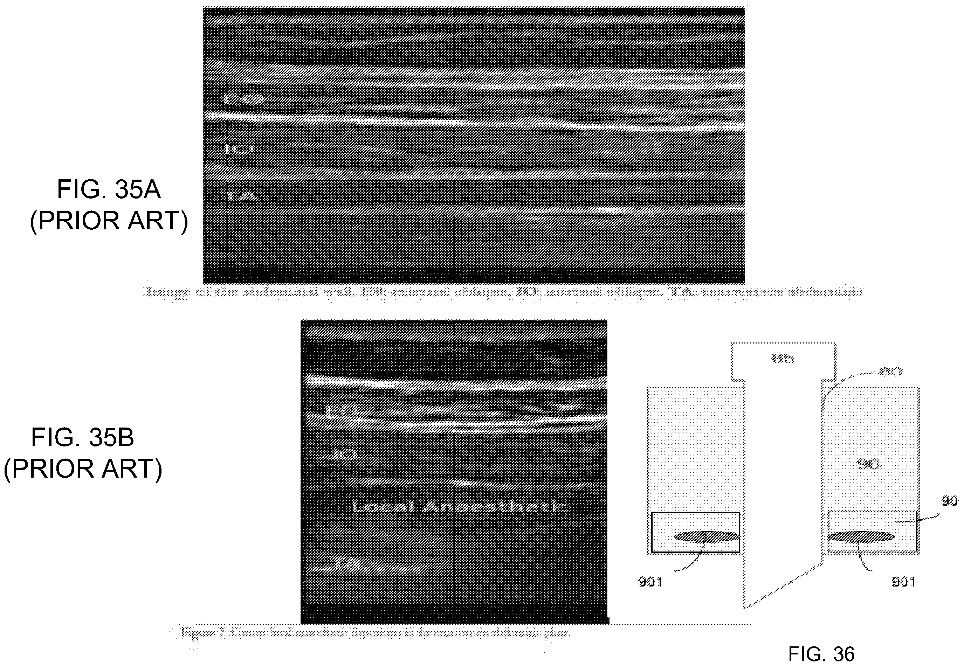

FIG. 35A is a prior art illustration, from above downwards, of skin; subcutaneous tissue; fat; and the external oblique (EO), internal oblique muscle (IO), the transversus abdominis (TA) muscles. The peritoneum and bowel loops may also be visualized deeper to the muscles.

FIG. 35B is a prior art illustration showing the desired positioning of a TAP block between the internal oblique muscle (IO), and the transversus abdominis (TA) muscles. Upon reaching the desired plane with ultrasound guidance, 2 ml of saline is injected to confirm correct needle position, after which 20 ml of local anesthetic solution is injected. The TAP plane is shown expanding with the injection, as indicated by the ultrasound.

In the present invention, an endoscopic trocar port closure device may be configured to inject or otherwise deliver one or more substances at a desired intra-muscular layer.

### SUMMARY OF INVENTION

The invention is defined in the appended claims.

A suture placement device is used to used to position a suture for intra-abdominal suturing and suturing of puncture wounds generated by surgical laparoscopic trocar ports and other puncturing devices.

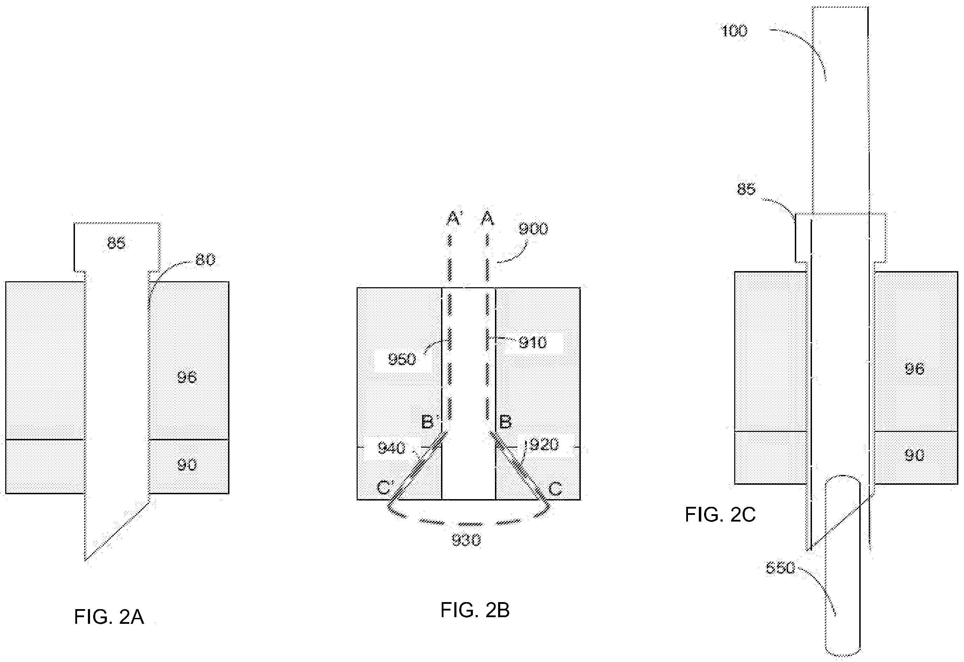

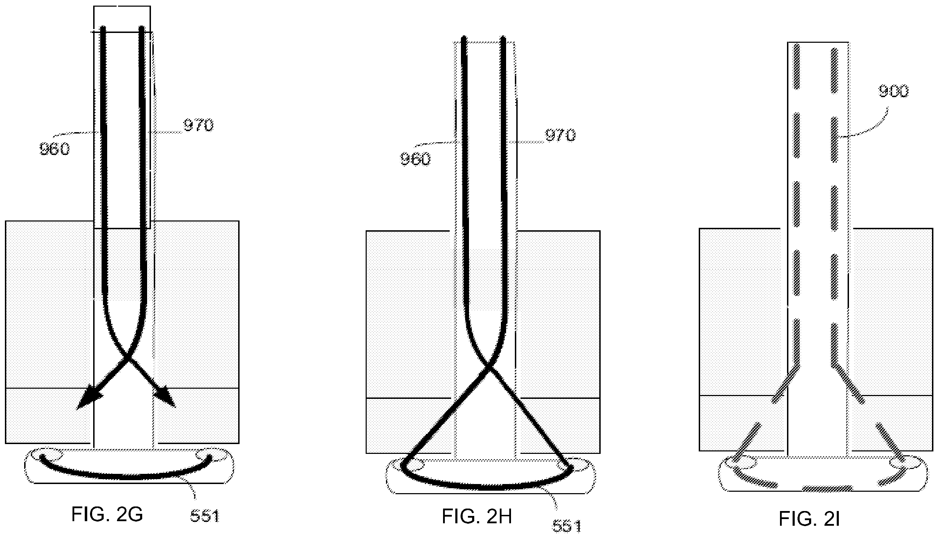

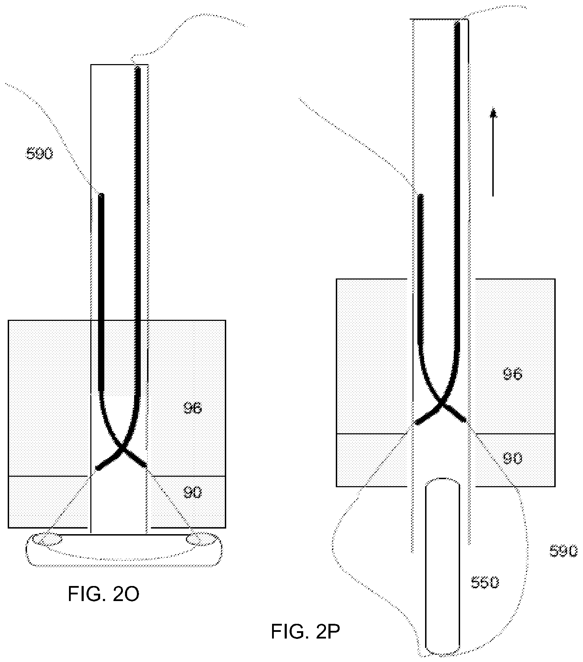

FIG. 2A is a cross section schematic of a trocar port **85** positioned in a trocar insertion site **80** through fat layer **96** and fascia **90.** When the trocar port is removed, it is desirable to close the insertion site by tying a suture with a knot which is tied above the fascia, and to have the suture pass through the abdominal wall about 1 cm from the edge of the insertion site or outside diameter of the trocar port.

FIG. 2B is a cross section schematic of the trocar insertion site **80** when the trocar port is removed. A desired suture **path A-B-C-C'-B'-A' 900** includes suture path segments **A-B 910, B-C 920, C-C' 930, C'-B' 940,** and **B'-A' 950** so that suture can be grasped at points **A** and **A'** in order to tie a knot.

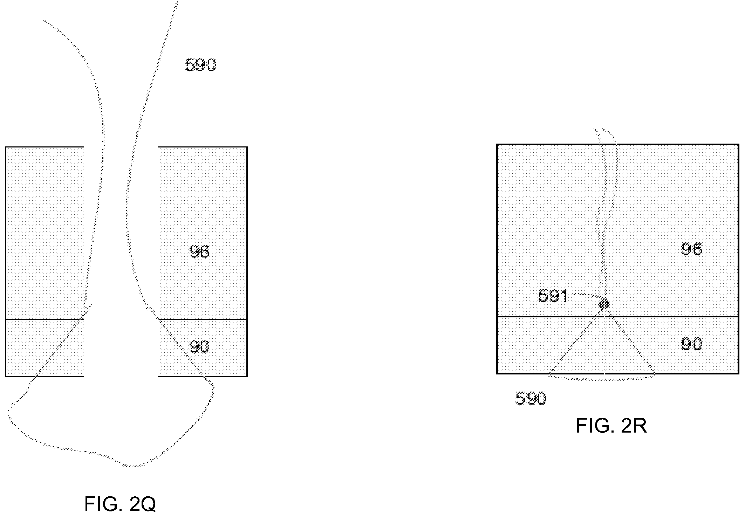

FIG. 2Q shows a suture **590** which has been routed through the desired suture path, and the suture placement device is withdrawn. FIG. 2R shows the suture **590** tied with a knot **591** so that the trocar insertion site is re-approximated. In order to create these path segments, it is necessary to remove the trocar port; to raise the bottom of the trocar port above **B** and **B';** or to provide slots or other access through the trocar port wall. In the examples below, the trocar port is removed after inserting a suture placement device, and the suture placement device is used to create a suture path so that a suture can be loaded into the path without loss of pneumoperitoneum. In other examples, the trocar may be partially raised, or slots may be provided in the trocar.

Prior art approaches typically use needles or suture passers to deliver a suture to points **C** and C', or to capture a pre-loaded suture from the proximity of points **C** and **C'.** In the embodiments described below, there are no exposed sharps, as the suture path is created and the suture is loaded through the suture path.

In the embodiments described below, suture path segment **C-C'** is created by providing an extension mechanism, such as a pivot bar or a pair of wings.

In the embodiments described below, suture path segments **B-C** and **B'-C'** are created by stylet guides which are deployed to penetrate the fascia and to provide stylet guide suture path segments between **B-C** and **B'-C'.**

In the example of FIGs 2A-2R, the extension mechanism is a pivot bar and the stylet guides are flexible guides which are deployed from within the housing of the suture placement device. Other structural elements and methods may be used to create the suture path segments, and several examples are described in this summary and in the detailed description.

In this example, a suture placement device **100** comprises an elongated body with its features in profile to allow use through a trocar port device **85** which can be removed over the suture placement device. The suture placement device comprises an elongated cannula having proximal and distal ends.

An extension mechanism, in this example a pivot bar, may be rotated from a first position aligned with the elongated cannula as shown in FIG. 2C, to a second extended position perpendicular to the elongated cannula.

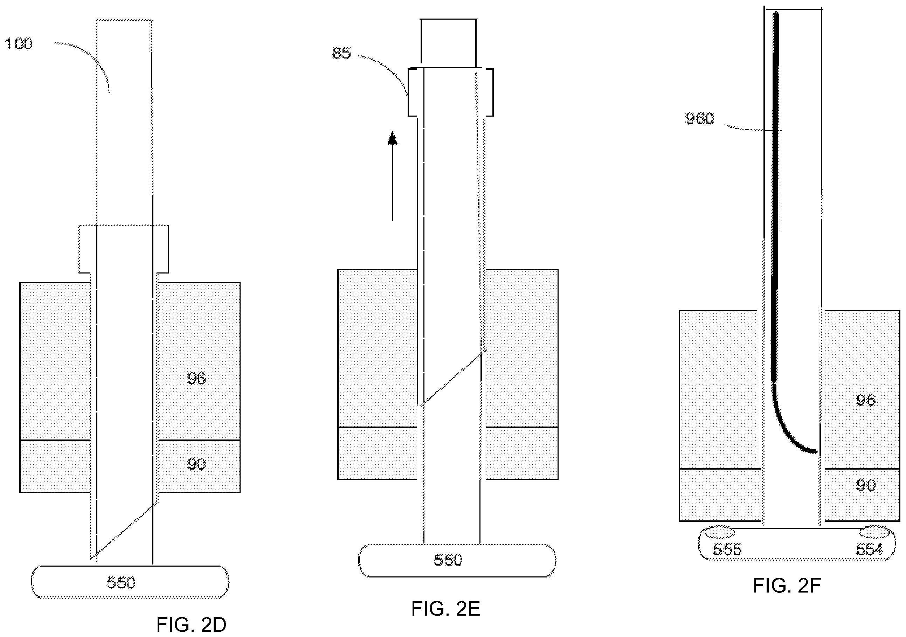

In the extended position as shown in FIGs. 2D-I, the upper surface of the pivot bar has a first receptor port **554** which is located a desired distance from the outside of the housing, typically 1 cm, and a diametrically opposed second receptor port **555** which is located a desired distance from the outside of the housing on the opposite side of the housing. A pivot bar suture path segment **551** is provided through the pivot bar between the first receptor port and the second receptor port.

The extended pivot bar permits the suture placement device to be held in place while the trocar port is removed, and protects the abdominal cavity from exposure to sharps.

A pair of hollow bore stylet guides **960** and **970** is provided in the housing. In a first, retracted position, the stylet guides are positioned in the housing. FIG. 2F illustrates a first stylet guide **960** in a retracted position. In a second deployed position, the stylet guides are pushed downwardly through the fascia and muscle until the lower ends of the stylet guides are pushed into the receptor ports.

FIG. 2G shows the stylet guides partially deployed through the fascia. A partially deployed position may be used to inject anesthetic or anti-inflammatory agents to a precise intra-muscular layer. In one example, the partially deployed position is established by a first mechanical stop on a plunger. In this example, the plunger and stop comprise the first injection tube depth positioning element. An analgesic, anti-inflammatory agent, or other substance may be injected through one or both partially deployed stylet guides. The stylet guides can then be fully deployed to complete the suture path and route a suture as described below.

When deployed as shown in FIG. 2H, the stylet guides provide stylet guide suture path segments. In this example, the stylet guides provide suture path segments from the receptor ports through the suture placement device, so that the combination of the stylet guide suture path segments and the pivot bar suture path segment provide a complete suture path **900.**

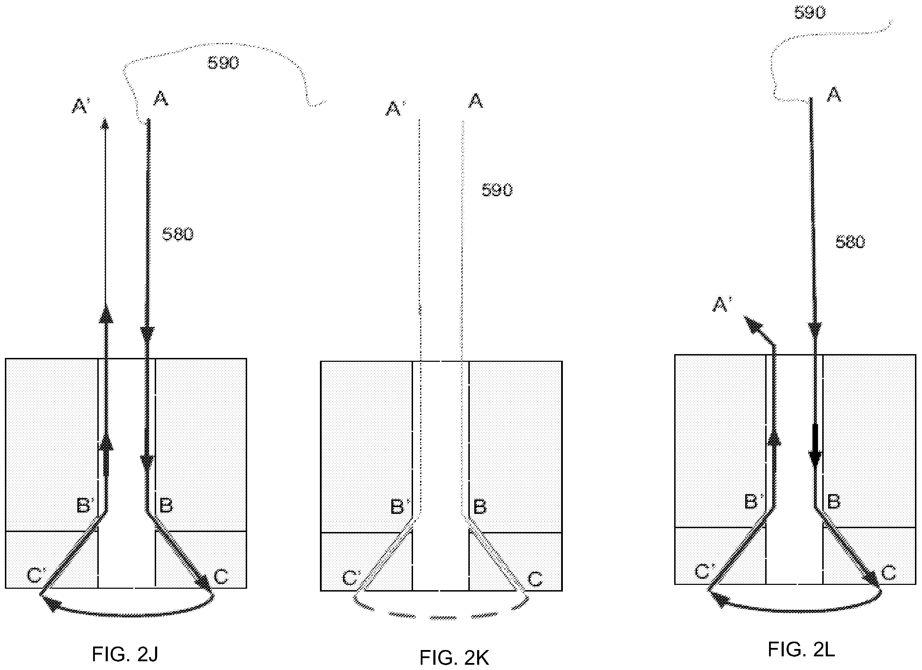

FIG. 2J illustrates a stylet **580** used to place a suture 590 in the suture path. In this example, one end of the suture is affixed to the distal end of a stylet; and the proximal end of the stylet is fed through a first stylet guide, down through the first receptor port, through the pivot bar suture path, up through the second stylet port, up through the second stylet guide and out of the housing. The first end of the stylet is then pulled out of the housing until the suture emerges from the housing, and the suture is removed from the second end of the stylet.

In FIG. 2J, the second stylet guide is routed through the proximal end of the device. In FIG. 2L, the second stylet guide is routed through the side of the device so that path **C-C'-B'-A'** is shorter than path **A-B-C.** This difference in length facilitates the use of a suture loading tool that has a leading flexible portion which is inserted into path **A-B-C;** and a plunger section which quickly drives the flexible portion through path **C-C'-B'-A'** as plunger is pushed through the path **A-B-C.**

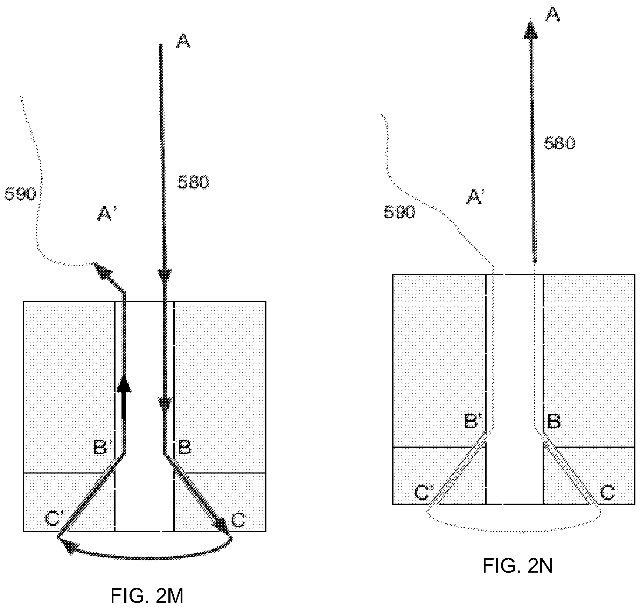

A suture may be carried with the leading edge of a stylet or suture loading tool, or the suture may be attached to the proximal end of the stylet and pulled backward through the suture path as illustrated by FIGs. 2M and 2N. The first end of the stylet is fed through the stylet guides and pivot bar suture path as described above; and the end of the suture is attached to the proximal end of the stylet when the proximal end emerges from the housing. The stylet is then pulled backward until the suture emerges from the proximal end of the first stylet guide, and the suture is removed from the first end of the stylet.

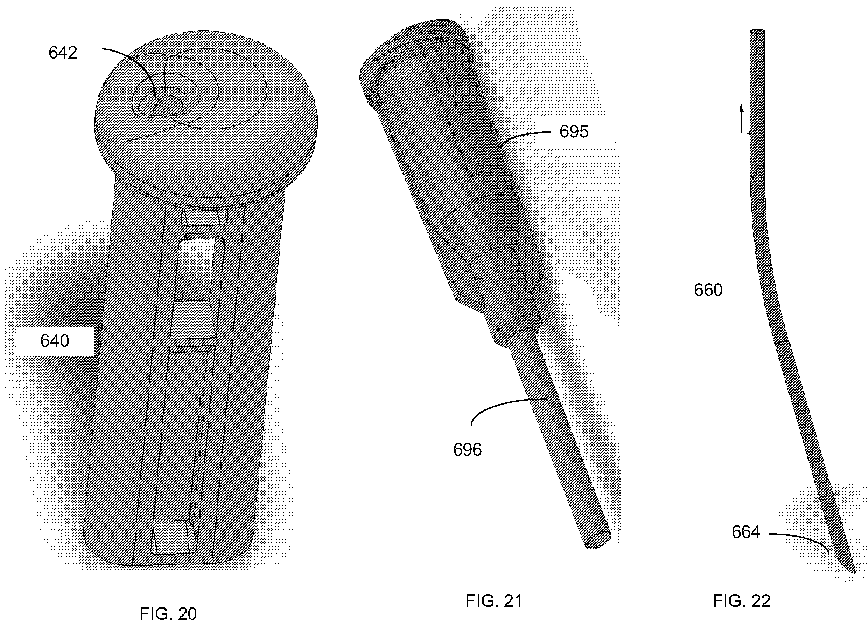

After the suture is routed, the stylet guides are retracted back into the housing, leaving portions of the suture in place in the fascia between the housing and the first receptor port, and between the housing and the second receptor port as illustrated in FIG. 20.

The pivot bar is rotated back to a vertical position as illustrated in FIG. 2P. A suture release slot is provided along the pivot bar suture path so that the suture is released from the pivot bar slot as the pivot bar is rotated back to alignment with the vertical axis of the cannula.

As the suture placement device is removed, the suture complex is thus deployed across the tissue defect site from within the abdominal cavity to secure the free ends of the suture outwardly from the inside of the peritoneal cavity via the laparoscopic port site. In one example, the device is configured to be utilized through a 10 mm or larger laparoscopic port, and is operative to position the suture at the tissue defect site such that the suture extends in a diametrically-opposed configuration at least 1 cm or greater across opposed edges of the tissue defect site which is caused by the laparoscopic trocar port device.

When the suture placement device is withdrawn from the laparoscopic trocar port site or other tissue defect site, the suture is securely attached to the innermost peritoneal cavity tissue layer, fascia, and muscle - incorporating at least 1 cm of the adjacent area surrounding the tissue defect site. Once the surgical suture(s) has been placed, a knot can be placed above the fascia to seal the tissue opening by re-approximating the edges of the defect created by the laparoscopic trocar device.

In the example above, the suture path segment **C-C'** is provided as a single path through an elongated pivotal element.

In other examples, the suture path segment **C-C'** may be established with a dual path technique where suture path segments extend from points **C** and **C'** to through the top of the device.

In the examples described below, a "stylet" is inserted into the suture path and used to pull an end of a suture through suture path segments.

In this specification, the term "stylet" means any mechanical element or assembly that is flexible and strong enough to be pushed or pulled through the suture path segments. In various examples, the "stylet" can be a classic configuration of a thin wire core within an outer wire loop; a leading section wire loop, with or without a thin wire cord, and a trailing plunger section with greater rigidity; and an assembly of a flexible leading portion, a more rigid plunger section, and a handle. The stylet may include features for grasping a portion of the suture.

### general method of suture placement

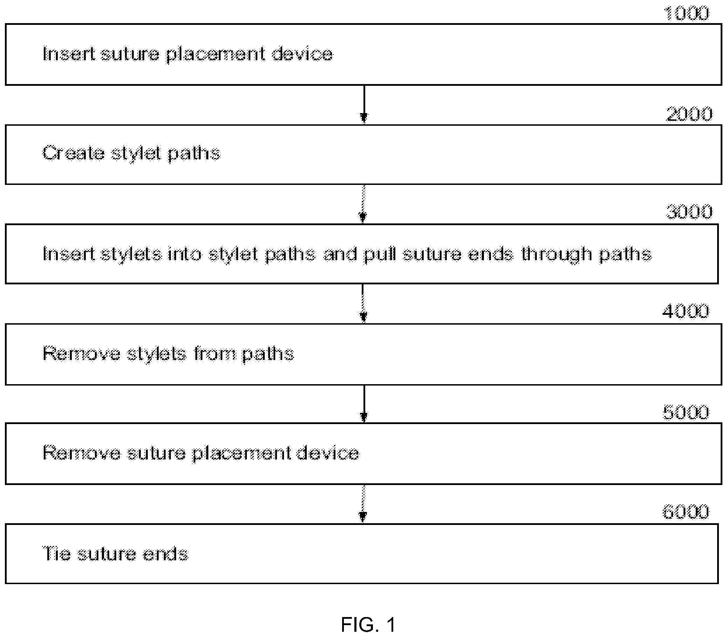

FIG. 1 is a flow chart which summarizes a general method of using a suture placement device to create suture paths and channels, and to use stylets to pull suture through the paths or channels.

At step **1000,** a suture placement device is provided in a cannular housing. In one embodiment, the suture placement device comprises a pair of deployable stylet guides which assist in creating suture paths or channels within the body tissue. A pivot bar or other extension mechanism is provided on the distal end of the suture placement device to prevent the deployable elements from piercing intestines or internal organs, and to establish a suture path below the fascia.

At step **2000,** a suture path is created.

In one embodiment, a single suture path is illustrated by FIGs. 2D and 2E:
- through the upper portion of the cannular housing down the first stylet guide, through the fascia to the first receptor port on the pivot bar,
- through the first receptor port, across the pivot bar, to the second receptor port on the pivot bar, and
- up through the second stylet guide through the fascia, and through the cannular housing.

The suture is not required to be preloaded into the cannular housing, and any desired suture can be used.

In the examples described below, the portions of suture path that is through fascia is created by penetrating the fascia in a downward direction with a flexible or rigid guide; or by penetrating the fascia in an upward direction by a curved guide, a telescoping curved guide, or a long flexible conduit.

At step **3000,** a stylet is inserted into the suture path, and the stylet is used to pull a suture through the suture path segments.

At step **4000,** the sty let is removed from the suture path. The suture falls free of the cannular housing as the suture placement device is removed, and the suture ends are tied to form a knot in or above the fascia in order to close a lower portion of the tissue opening.

At step **5000,** the suture placement device is removed. In one embodiment, stylet guides are retracted back into the cannular housing and a pivot bar element is rotated back to a retracted position before the suture placement device is removed.

At step **6000,** the suture ends are tied.

### method of modifying port site closure device to provide TAP block

Prior art methods, such as the ultrasound-guided approach described above, attempt to locate the desired injection site by measuring from above the site. Variations between patients' fat layer thickness make it difficult to know the exact depth of the desired injection site.

While fat layer thickness varies substantially between patients, the distance between the abdominal wall and the desired injection site is much more constant. A fascial plane reference element such as wings, a pivot bar, needle guides, or other deployable element can locate the abdominal wall so that an injection depth positioning device can properly position one or more injection tubes for delivering a substance to a desired inter-muscular layer.

In one embodiment, the injection depth positioning device comprises a fascial plane reference element such as a pivot bar or wings; a first injection tube (stylet guide) having a distal end; and a first injection tube depth positioning element. These elements can be provided in suture placement devices.

Examples of injection tube depth positioning elements include fixed mechanical stops, adjustable mechanical stops; and visual indicators such as color bands and scales.

In some examples, a suture placement device facilitates the placement or injection of a substance or substances in proximity to a trocar port at a controlled depth.

FIG. 33 is a flow chart which summarizes a general method of modifying a port site closure device to inject a substance at a desired depth within the fascia muscle layers.

At step 1050, a modified port closure device is provided. In one embodiment, the modified port closure device comprises a housing; a fascial plane reference element; a first injection tube having a distal end; a first injection tube depth positioning element; and a pair of deployable stylet guides which assist in creating suture paths or channels within the body tissue.

At step 1100, the modified port closure device is inserted into a trocar port or into a trocar port site after a trocar port has been removed.

At step 1200, the trocar is removed if it has not been previously removed.

At step 1300, the location of the facial plane is established with the fascial plane reference element of the modified port closure device. In various examples, the fascial plane reference element is a pivotal bar, one or more wings, fixed hooks, needle guides, or other element.

At step 1400, the injection tube depth positioning element is used to position the injection tube distal end at a first tissue depth relative to the location of the fascial plane. In various examples, the injection tube depth positioning element is a fixed mechanical stop; an adjustable mechanical stop; color coded depth bands; an alphanumeric scale; an encoder and signal; or other element.

At step 1500, the substance is injected or otherwise introduced through the injection tube. In various examples, the injection tube is a flexible stylet guide, a fixed stylet guide, a fixed straight needle, a flexible needle, a curved needle, a telescoping guide or needle, or other element. The injection tube may serve a dual purpose as a suture passer or stylet guide.

In various examples, a single injection tube may be used to make a single injection or multiple injections, or multiple injection tubes may be used.

The injection tube may be positioned at a second tissue depth, such as to place a suture or to position a stylet guide.

### Suture placement devices

In one embodiment, a single suture path is created by a pair of stylet guides which are deployed downwardly and outwardly from two sides of a cannular housing. These guides are designed to penetrate tissue by having a sharp bevel-shaped tip and are designed to create a suture path segment for a stylet by having a hollow-bore tunnel built within the guide.

An extension mechanism, a pivot bar, is deployed from the housing, and positioned below the fascia. A channel is provided in the pivot bar, and extends from a receptor port on each side of the pivot bar, such that the receptor ports are located about 1 cm from opposed sides of the outside of the cannular housing. When the stylet guides are fully deployed, the distal end of each stylet guide engages a receptor port, thereby creating a suture path through the first stylet guide, into the first receptor port, through the channel, through the second receptor port, and through the second stylet guide. A stylet is then directed through the suture path to pull the first end of a suture through the path. After routing the suture, the stylet guides are retracted, leaving the suture routed through the fascia. The pivot bar has a suture release slot, and the suture is released through the suture release slot as the pivot bar is rotated back to a folded position. The suture placement device is withdrawn and the suture is tied.

One or both stylet guide may have a partially deployed position so that the distal end of the guide is precisely positioned relative to the fascia. An anesthetic or anti-inflammatory agent is injected through one or both guides to improve post-surgery comfort of a patient.

In one example, the stylet guides have openings at the proximal end of the housing. In another example, a first stylet guide has an entry opening at the proximal end of the housing, and a second stylet guide has an exit opening below the proximal end of the housing so that the exit path is shorter than the inlet path.

In one example, the device is a disposable plastic device. In another example, the device is a reusable metal or plastic device. In other examples, the device comprises a re-usable metal housing and a disposable guide assembly which may include a replaceable pivot bar.

In this example, the pivot bar serves as a fascial plane reference element and one or both stylet guides serves as an injection tube. The desired depth of the distal end of the stylet guide(s) may be set with a fixed mechanical stop or an adjustable mechanical stop, or manually with the help of a user guide such as one or more color coded bands or a ruling. A single mechanical plunger may be used to set the depth of both stylet guides.

In one example, the stylet guides have openings at the proximal end of the housing. In another example, a first stylet guide has an entry opening at the proximal end of the housing, and a second stylet guide has an exit opening below the proximal end of the housing so that the exit path is shorter than the inlet path. A luer fitting or a-ring may be provided on the entry opening so that a syringe may be used to inject a substance through the first stylet guide.

### rigid stylet guides aligned by housing

In this example, an extension mechanism, such as a pivot bar or pair of wings, is deployed as described above to position receptor ports below the fascia. A suture path segment is provided between the receptor ports.

A pair of rigid stylet guides are deployed at a downward angle through alignment slots provided in the housing. The guides penetrate tissue and create a suture path segment for a stylet. After routing the suture, the stylet guides are retracted, leaving the suture routed through the fascia. The suture is released from the housing alignment slot by suture release slots provided on the housing, and released from the extension mechanism as the pivot bar or wings are rotated back to a folded position and the suture placement device is withdrawn.

One or both stylet guide may have a partially deployed position so that the distal end of the guide is precisely positioned relative to the fascia. An anesthetic is injected through the guide(s) to improve post-surgery comfort of a patient.

In these examples, the pivot bar or v,rings serve as a fascial plane reference element and one or both stylet guides serves as an injection tube. The desired depth of the distal end of the stylet guide(s) may be set with a fixed mechanical stop or an adjustable mechanical stop, or manually with the help of a user guide such as one or more color coded bands or a ruling.

### DESCRIPTION OF FIGURES

These and other features, aspects, and advantages of the present invention will become better understood with regard to the following description, appended claims, and accompanying drawings where:
FIG. 1 is a flow chart that summarizes a general method of using a suture placement device to create suture paths, and to use one or more stylets to pull a suture through the paths.
FIG. 2A is a cross section schematic of a trocar port positioned in a trocar insertion site though fat layer and fascia.
FIG. 2B is a cross section schematic of a trocar insertion site showing ideal suture paths through the fascia.
FIG. 2C is a cross section schematic of a suture placement device partially inserted in to the trocar port oLFlG. 2A with a pivot bar in a folded position.
FIG. 2D is a cross section schematic of the suture placement device of FIG. 2C fully inserted in to the trocar port, and the pivot bar in an extended position.
FIG. 2E is a cross section schematic of the suture placement device of FIG. 2D, with the trocar port being removed over the device.
FIG. 2F is a cross section schematic of the suture placement device of FIG. 2E pulled upward against the abdominal wall.
FIG. 2G is a cross section schematic of the suture placement device of FIG. 2E showing a distal suture path through the pivot bar, and stylet guides partially deployed.
FIG. 2H is a cross section schematic of the suture placement device of FIG. 2E showing stylet guides fully deployed.
FIG. 21 is a cross section schematic of the suture placement device of FIG. 2H showing stylet path segments.
FIG. 2J is a cross section schematic of the suture placement device of FIG. 21 showing a stylet routed through the stylet path segments.
FIG. 2K is a cross section schematic of the suture placement device of FIG. 2J showing a suture pulled through the stylet path segments.
FIG. 2L is a cross section schematic of the suture placement device of FIG. 21 showing a stylet routed through the stylet path segments with an alternative exit path.
FIG. 2M is a cross section schematic of the suture placement device of FIG. 2L showing a suture attached to the stylet.
FIG. 2N is a cross section schematic of the suture placement device of FIG. 2M showing the suture pulled through the suture path.
FIG. 20 is a cross section schematic of the suture placement device of FIG. 2M showing the stylet guides retracted.
FIG. 2P is a cross section schematic of the suture placement device of FIG. 20 showing the pivot bar folded and the suture released from the pivot bar.
FIG. 2Q is a cross section schematic of the suture placement device of FIG. 2P removed, leaving the suture in place.
FIG. 2R is a cross section schematic of the suture tied to reapproximate the trocar port opening.

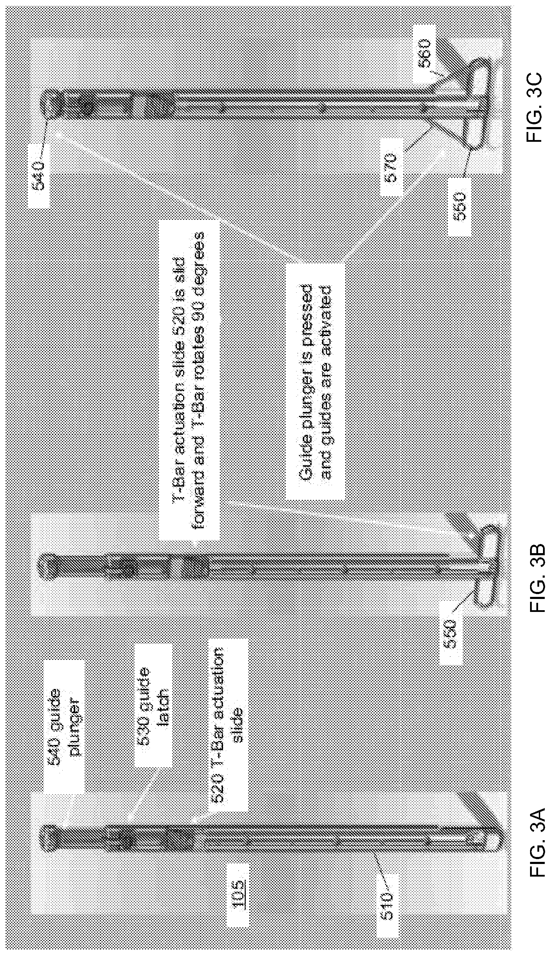

FIG. 3A is a side perspective view of another embodiment of a suture placement device.
FIG. 3B is a side perspective view of the embodiment of the suture placement device of FIG. 3A with a pivot bar wing deployed.
FIG. 3C is a side perspective view of the embodiment of the suture placement device of FIG. 3A with a first and second guide deployed to engage the pivot bar wing.

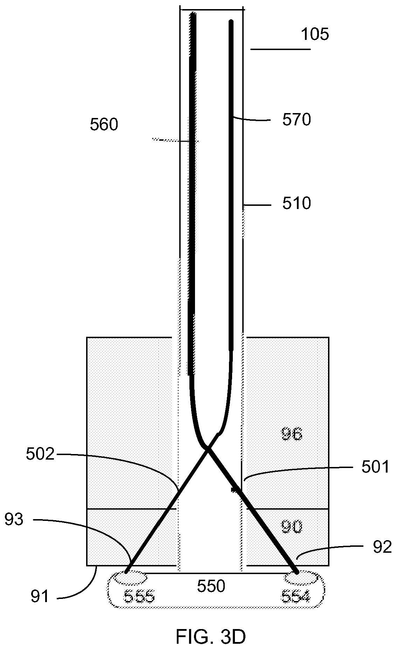

FIG. 3D shows the suture placement device of FIG. 3A inserted into tissue fat and fascia layers.

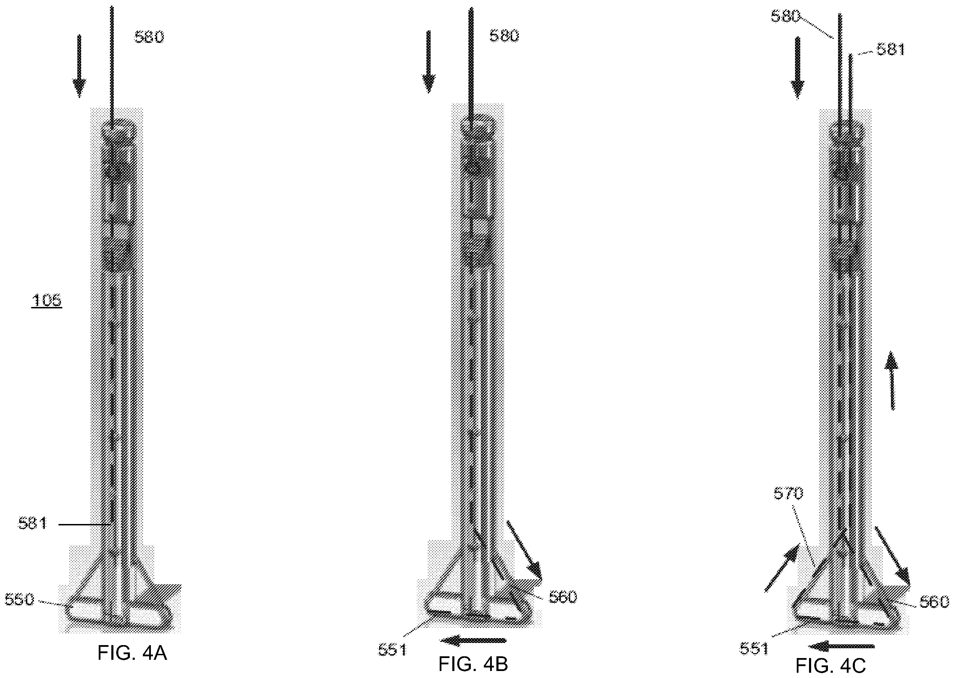

FIG. 4A is a side perspective view showing a stylet partially inserted into the first guide of the embodiment of the suture placement device of FIG. 3A.
FIG. 4B is a side perspective view showing a stylet inserted into the first guide and pivot bar channel of the embodiment of the suture placement device of FIG. 3A.
FIG. 4C is a side perspective view showing a stylet inserted into the first guide, pivot bar channel, and second guide of the embodiment of the suture placement device of FIG. 3A.

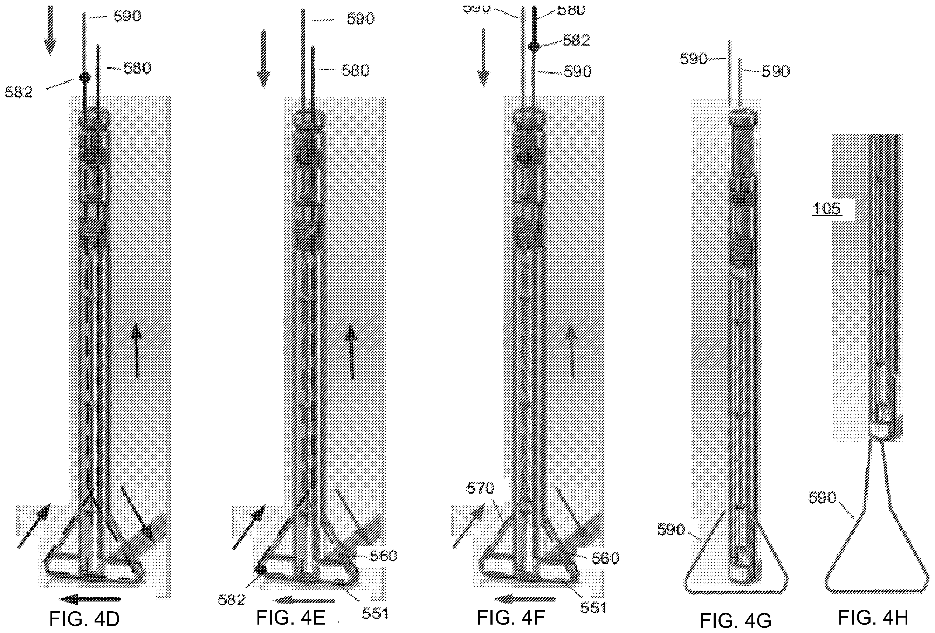

FIG. 4D is a side perspective view showing a stylet pulling the end of a suture into the first guide of the suture placement device of FIG. 3A.
FIG. 4E is a side perspective view showing a stylet pulling the end of a suture through the first guide and channel of the suture placement device of FIG. 3A.
FIG. 4F is a side perspective view showing a stylet pulling the end of a suture through the first guide, channel, and second guide of the suture placement device of FIG. 3A.
FIG. 4G is a side perspective view showing a suture positioned through the first guide, channel, and second guide of the suture placement device of FIG. 3A, and the pivot bar wing in a retracted position.
FIG. 4H is a side perspective view showing the suture of FIG. 3G as the suture placement device is partially removed.

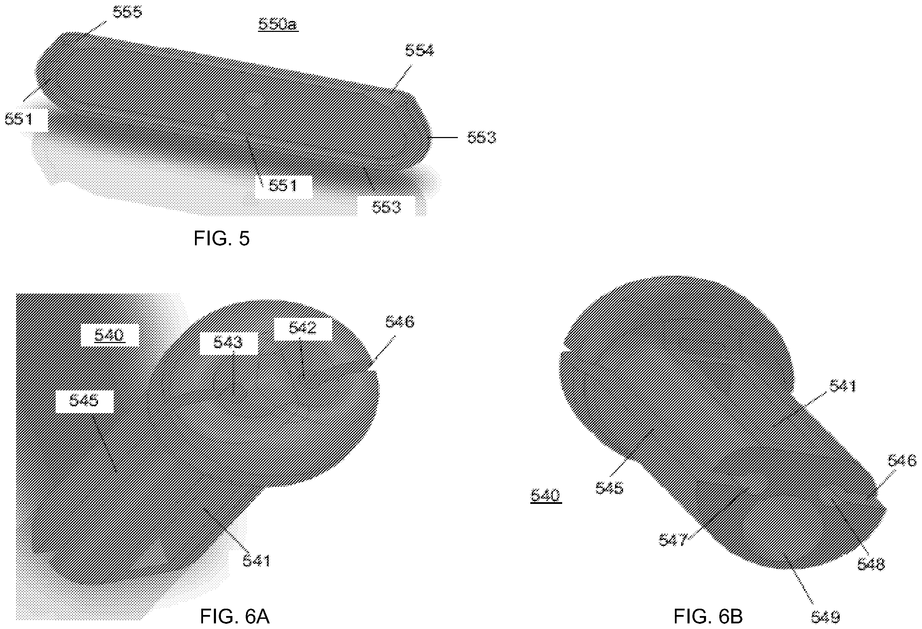

FIG. 5 is a side perspective view showing a first half of a pivot bar wing for the suture placement device of FIG. 3A.
FIG. 6A is a top perspective view of a plunger for the suture placement device of FIG. 3A.
FIG. 6B is a bottom perspective view of the plunger of FIG. 6A.

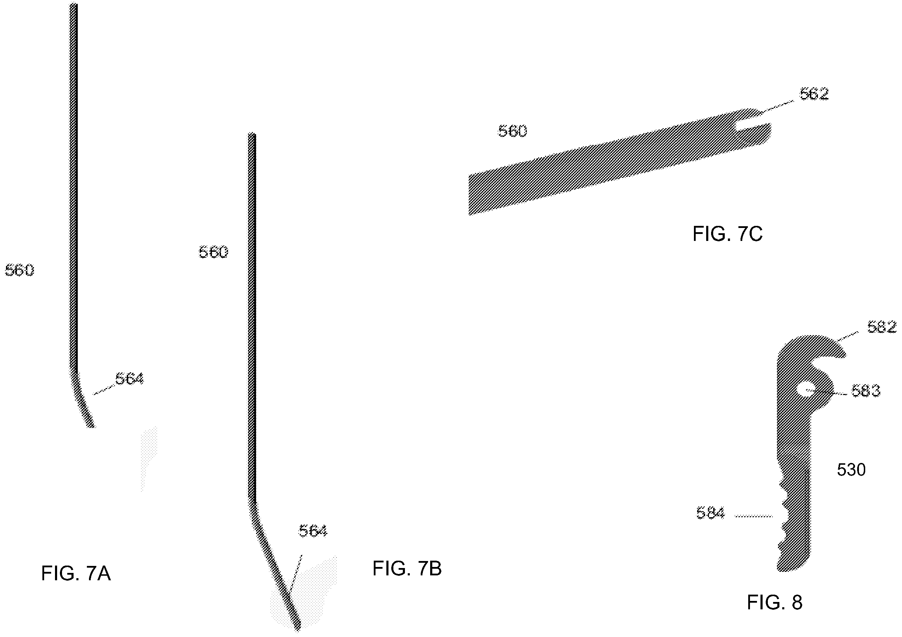

FIG. 7A is a side view of a first guide for the suture placement device of FIG. 3A with the guide in a retracted position.
FIG. 7B is a side view of a first guide for the suture placement device of FIG. 3A with the guide in an extended position.
FIG. 7C is a detailed side view of an end of the first guide of FIG. 6A showing a slot on the guide.
FIG. 8 is a side view of a guide latch for the suture placement device of FIG. 3A.

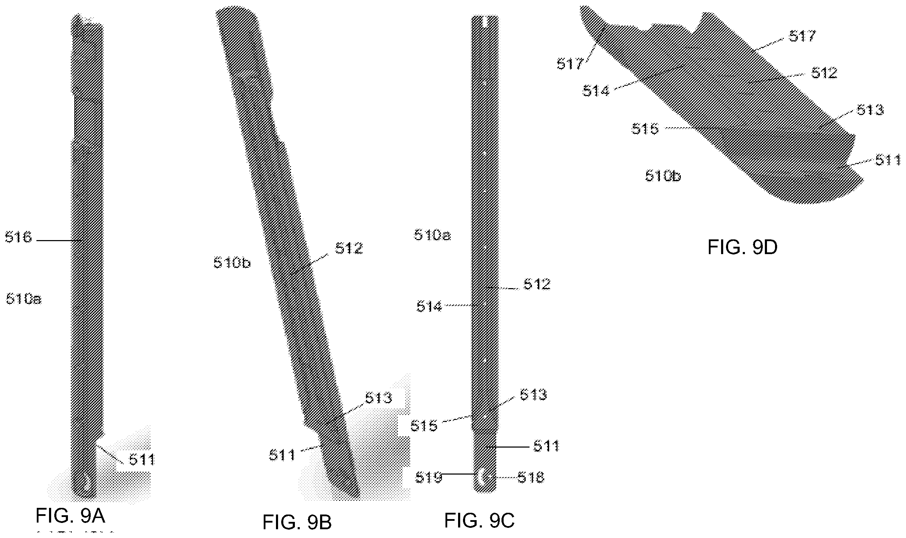

FIG. 9A is a side perspective view of the outside of a first body section for the suture placement device of FIG. 3A.
FIG. 9B is a side perspective view of the inside of a second body section for the suture placement device of FIG. 3A.
FIG. 9C is a side view of the inside of the first body section of FIG. 9B.
FIG. 9D is a bottom perspective view of the inside of the second body section of FIG. 9A.

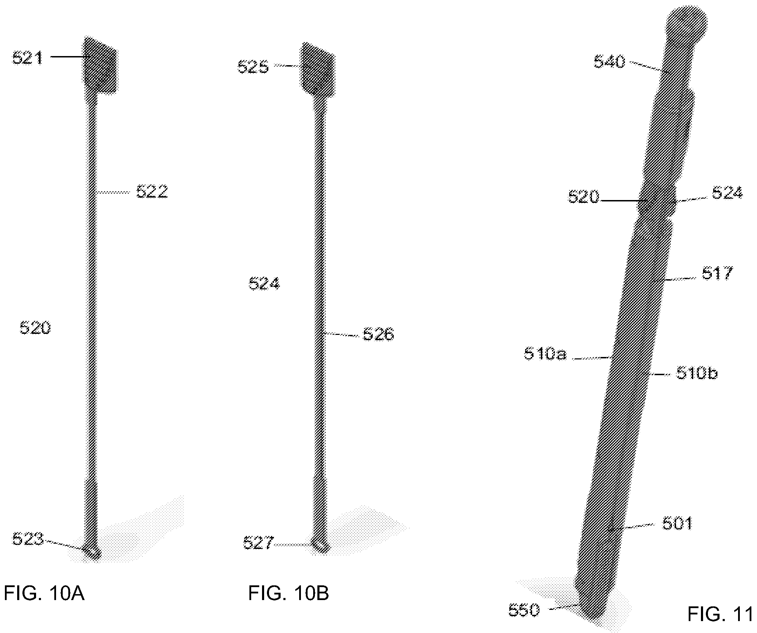

FIG. 10A is a side perspective view of a first actuation arm for the suture placement device of FIG.3A.
FIG. 10B is a side perspective view of a second actuation arm for the suture placement device of FIG.3A.
FIG. 11 is a side perspective view of the suture placement device of FIG. 3A with the pivot bar wing in a retracted position.

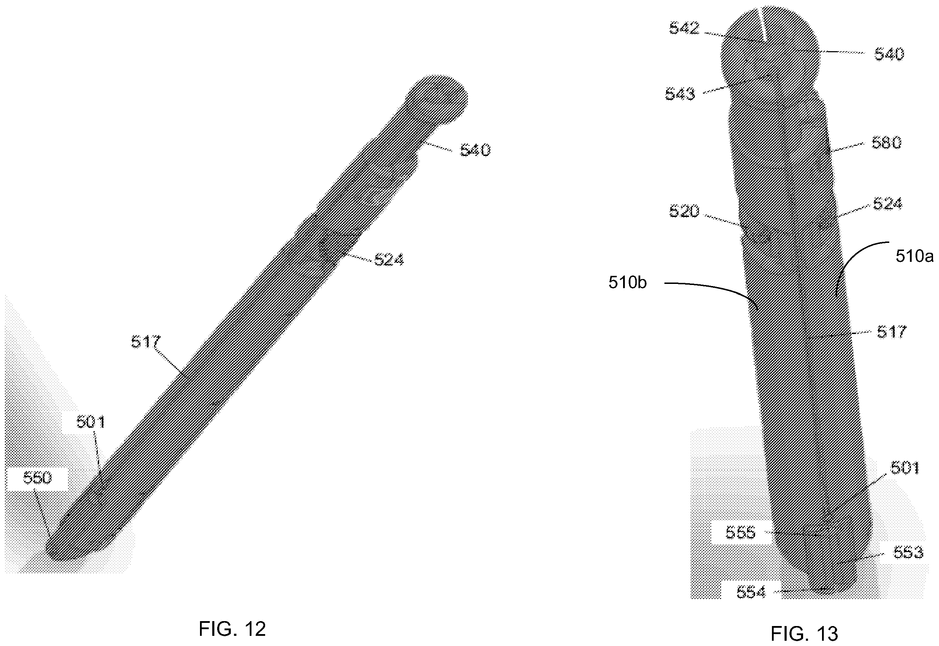

FIG. 12 is a side perspective view of the suture placement device of FIG. 3A with the pivot bar wing in a retracted position.
FIG. 13 is a top perspective view of the suture placement device of FIG. 3A with the pivot bar wing in a retracted position.

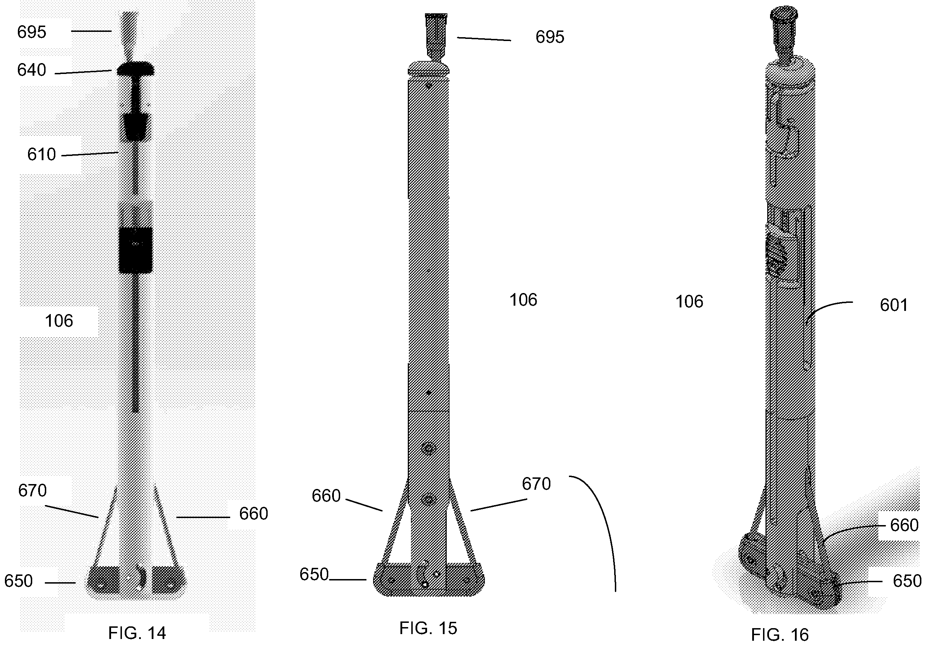

FIG. 14 is a front view of a plastic suture placement device.
FIG. 15 is a rear view of the suture placement device of FIG. 14.
FIG. 16 is a side perspective view of the suture placement device of FIG. 14.

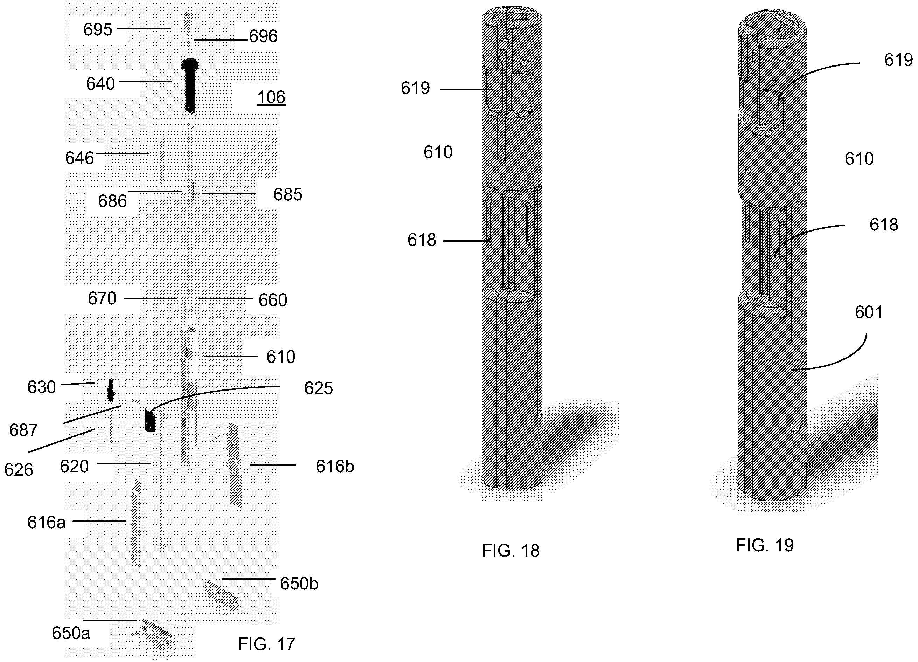

FIG. 17 is an exploded side perspective view of the suture placement device of FIG. 14.
FIG. 18 is a front view of the upper body portion of the plastic suture placement device of FIG. 14.
FIG. 19 is a side perspective view of the upper body portion of the plastic suture placement device of FIG. 14.
FIG. 20 is a top perspective view of the plunger of the plastic suture placement device of FIG. 14.
FIG. 21 is a side perspective view of a luer fitting and extension tube which may be inserted into the plastic suture placement device of FIG. 14.
FIG. 22 is a front view of stylet guides of the suture placement device of FIG. 14.

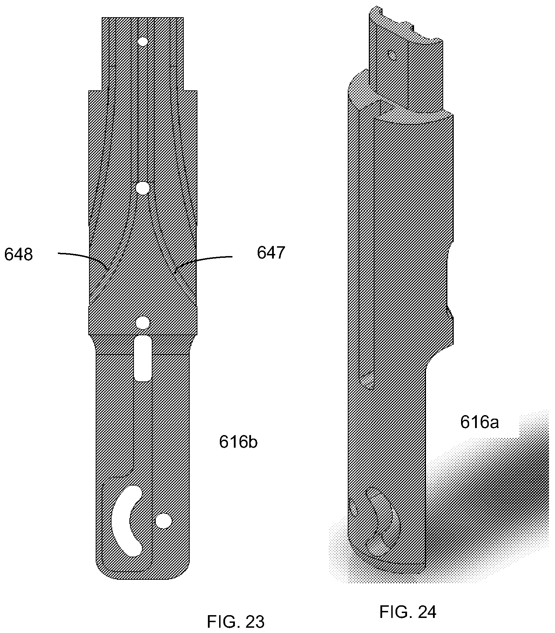

FIG. 23 is a front view of the interior of lower body section of the suture placement device of FIG. 14.
FIG. 24 is a front perspective view of the exterior of lower body section of the suture placement device of FIG.14.

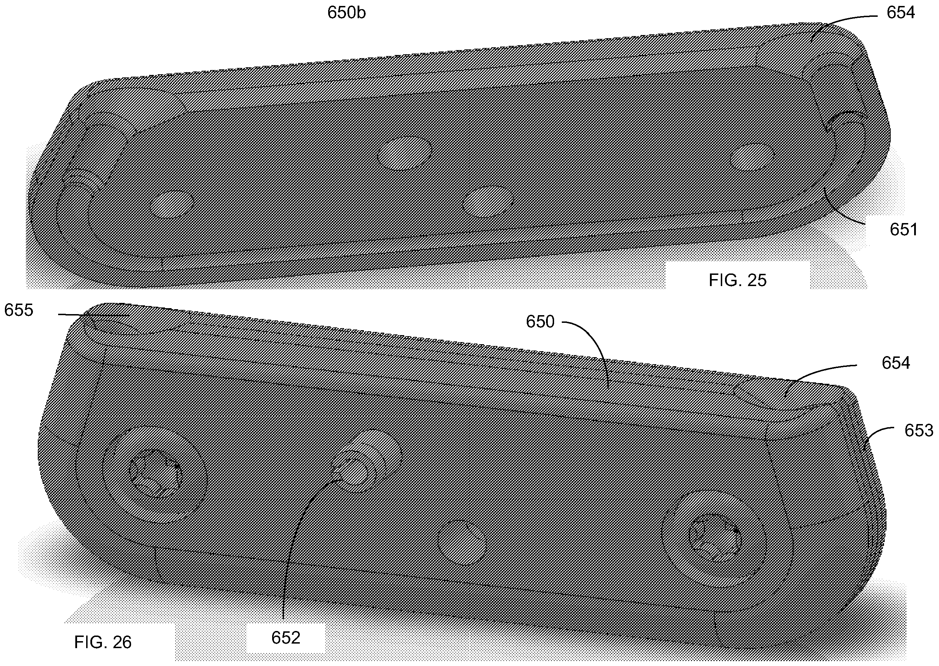

FIG. 25 is a front perspective view of a pivot bar section of the suture placement device of FIG.14.
FIG. 26 is a front perspective view of an assembled pivot bar of the suture placement device of FIG.14.

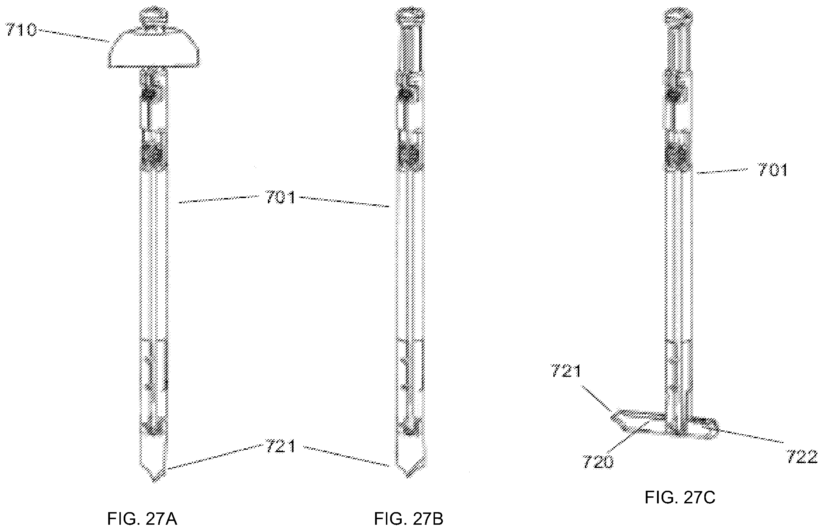

FIG. 27A shows a suture placement device that is configured to be used as an obturator.
FIG. 27B shows the suture placement device/obturator of FIG. 27A with the head removed so that the device is configured to be used as a suture placement device.
FIG. 27C shows the pivot bar 720 deployed when the device of FIG. 27B is inserted into a trocar port.

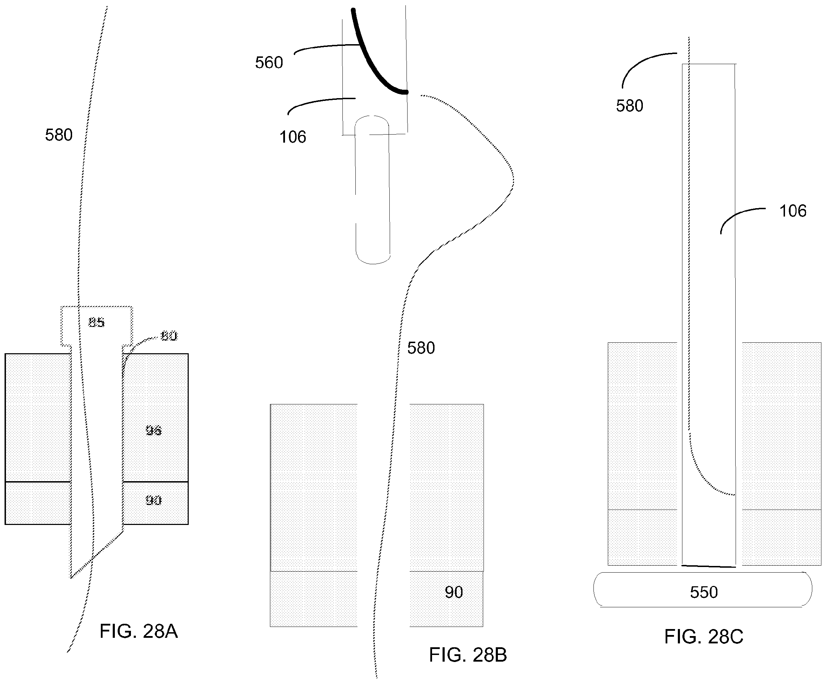

FIG. 28A is a cross section view of a stylet inserted into a 8mm trocar port.
FIG. 28B is a cross section view of the stylet of FIG. 28A inserted into a stylet guide of a suture placement device.
FIG. 28C is a cross section view of the stylet of FIG. 28A withdrawn into the stylet guide of the suture placement device of FIG. 28B.

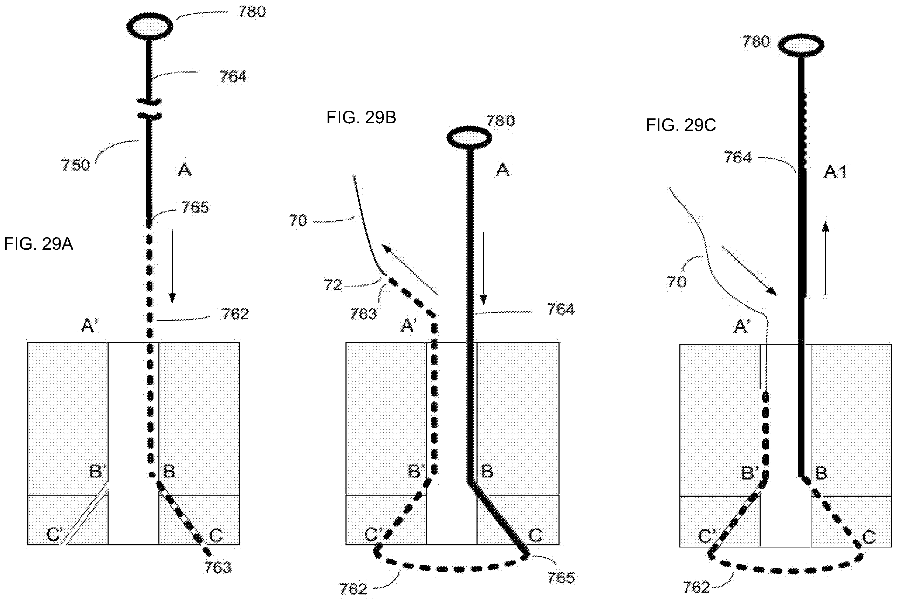

FIG. 29A is a cross section illustration of a portion of a suture loading device partially inserted into a suture placement device.
FIG. 29B is a cross section illustration of the suture loading device of FIG. 29A fully inserted into a suture placement device with a suture attached to the distal end of the suture loading device.
FIG. 29C is a cross section illustration of the suture loading device of FIG. 29A partially withdrawn from the suture placement device.

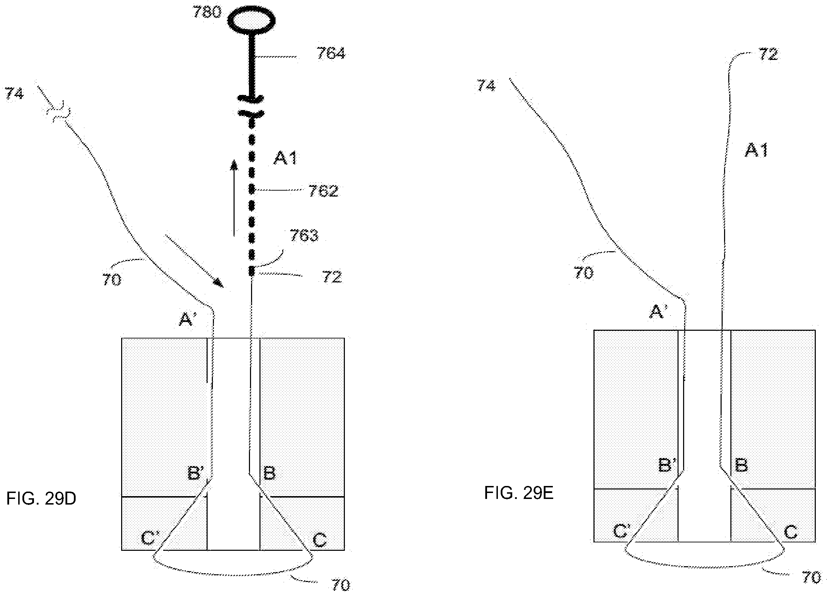

FIG. 29D is a cross section illustration of the suture loading device of FIG. 29A almost folly withdrawn from the suture placement device.
FIG. 29E is a cross section illustration of the suture loading device of FIG. 29A fully withdrawn from the suture placement device and detached from the suture.

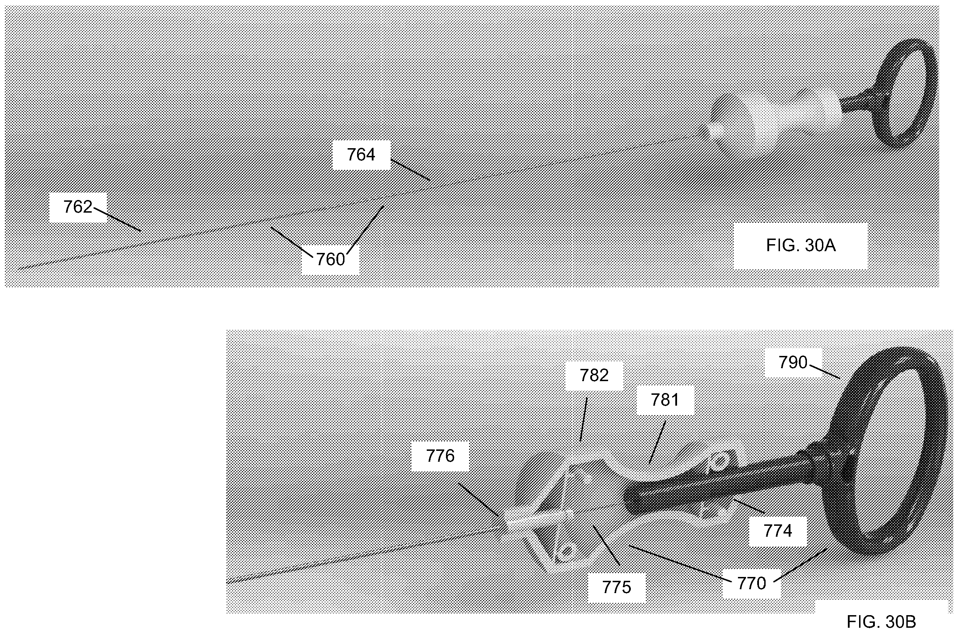

FIG. 30A is a front perspective view of a suture loading device.
FIG. 30B is a detailed cross section view of a handle assembly of the suture loading device of FIG.30A.

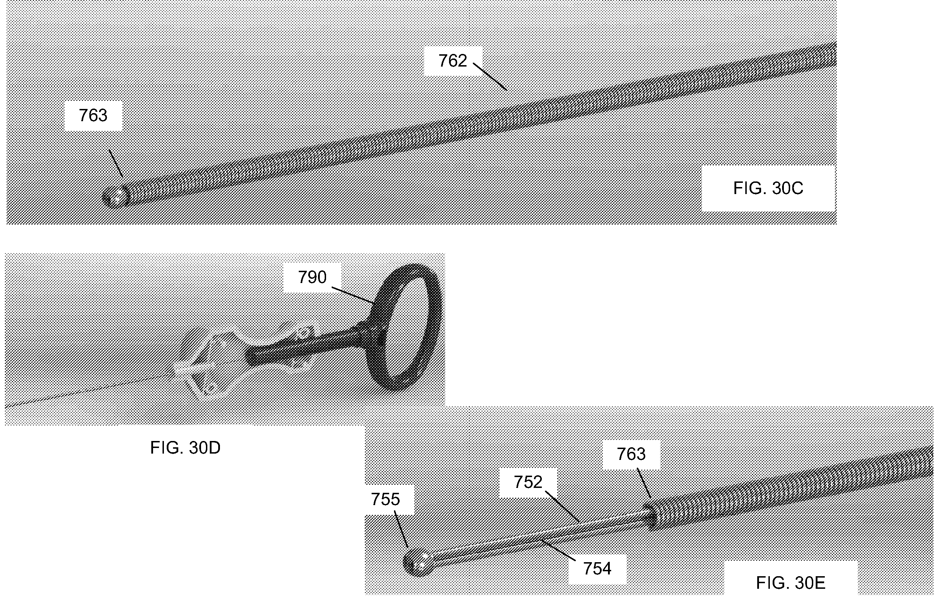

FIG. 30C is a detailed view of the distal end of the suture loading device of FIG. 30A in its normally closed orientation.
FIG. 30D is a detailed cross section view of a handle assembly of the suture loading device of FIG. 30A in a suture capture/release orientation.
FIG. 30E is a detailed view of the distal end of suture loading device of the suture loading device of FIG. 30A with the device in a suture-capture orientation.

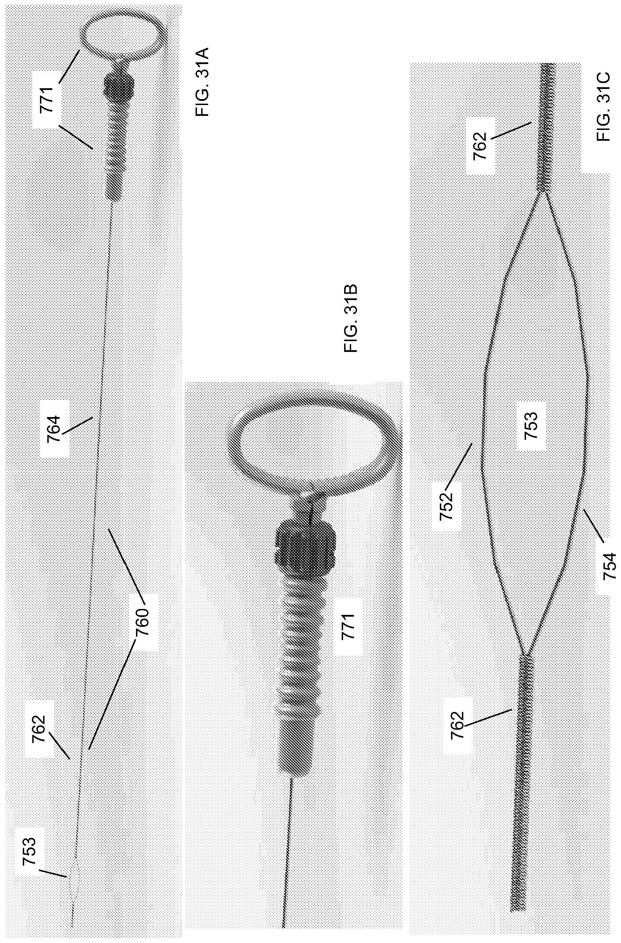

FIG. 31A is a front perspective view of another suture loading device.
FIG. 31B is a detailed cross section view of a handle assembly of the suture loading device of FIG. 31A.
FIG. 31C is a detailed view of the distal end portion of the suture loading device of FIG. 31A.

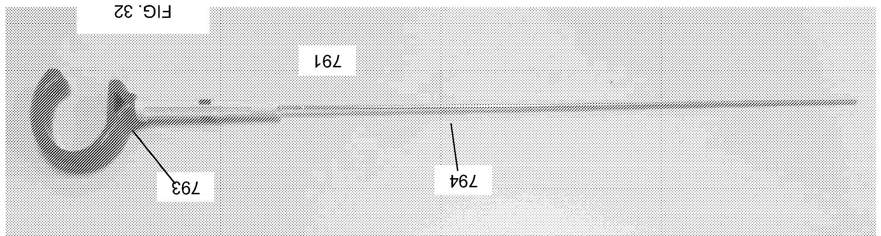

FIG. 32 is a front perspective view of a plunger suture loading device.
FIG. 33 is a flow chart that summarizes a general method for injecting a substance at a desired depth within the fascia.
FIG. 34 is a prior art illustration of a TAP block.
FIG. 35A is a prior art illustration of skin; subcutaneous tissue; fat; and the external oblique (EO), internal oblique muscle (IO), the transversus abdominis (TA) muscles.
FIG. 35B is a prior art illustration showing the desired positioning of a TAP block between the internal oblique muscle (IO), and the transversus abdominis (TA) muscles.
FIG. 36 is a cross section view of a trocar port and trocar port site showing desired injection plane for TAP block.

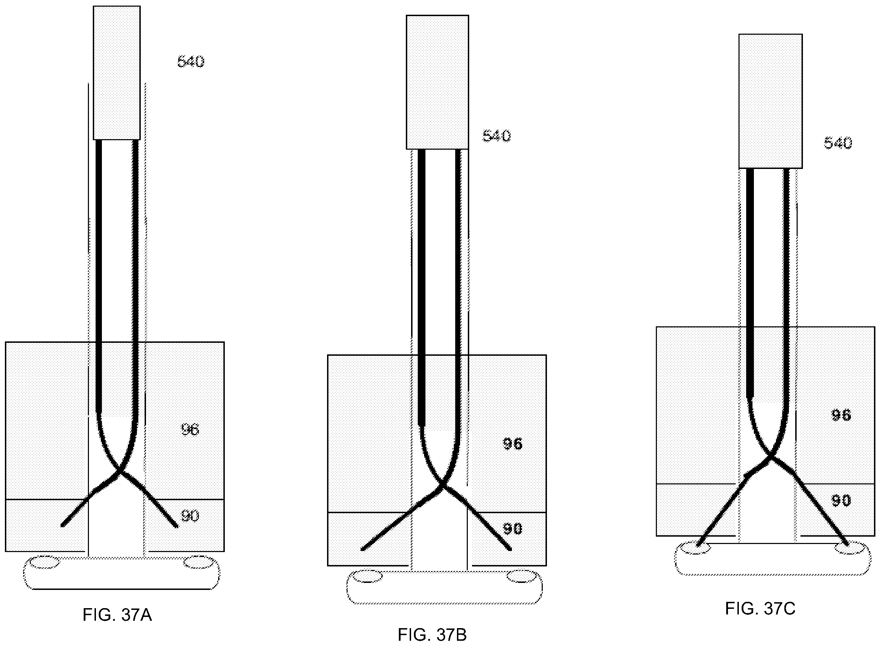

FIG. 37A shows a guide plunger for a suture placement device partially depressed so that stylet guide tips are positioned at a first distance from the fascial plane reference element.
FIG. 37B shows the guide plunger of FIG. 37A partially depressed so that the stylet guide tips are positioned at an optional second distance from the fascial plane reference element.
FIG. 37C shows the guide plunger of FIG. 37A fully depressed so that the stylet guide tips are fully deployed.

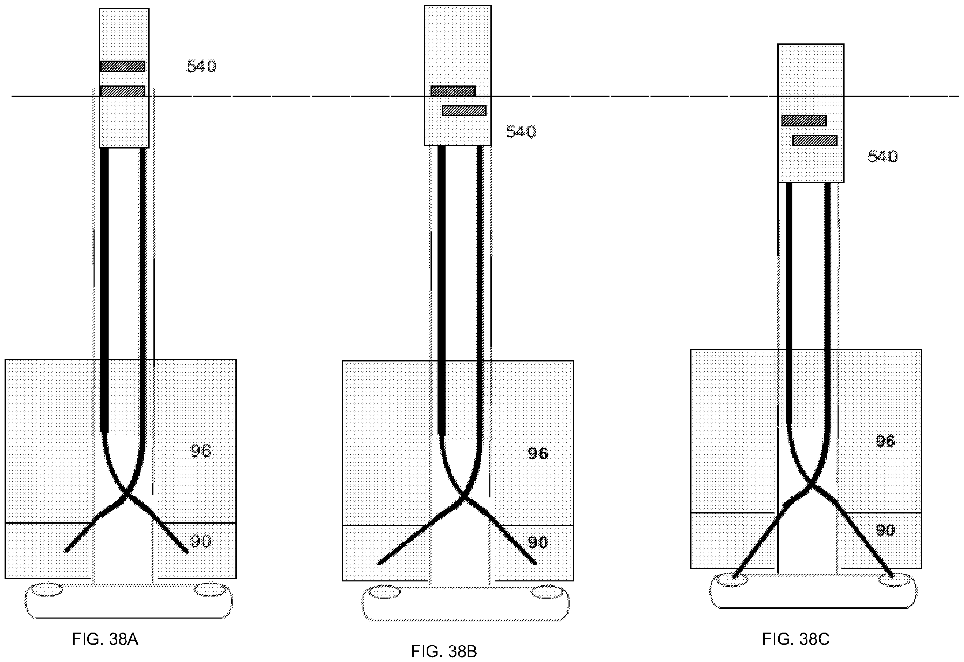

FIG. 38A shows shows a guide plunger partially depressed according to a first color band or scale reading on the housing.
FIG. 38B shows the guide plunger partially depressed according to a second color band or scale reading on the housing.
FIG. 38C shows the guide plunger fully depressed so that the stylet guide tips are fully deployed.

### DESCRIPTION OF EMBODIMENT- suture placement device with pivot bar and stylet guides deployed downwardly from housing

In this embodiment, a pair of flexible stylet guides are deployed downwardly and outwardly from opposite sides of a cannular housing. The distal end of each guide engages a receptor port on a pivot bar element which is deployed from the housing. The stylet guides penetrate tissue with sharp bevel shaped tips that create a suture path segment for a stylet through the hollow guide. When engaged in the receptor ports, the stylet guides hold the suture placement device in position so that the trocar port can be removed over the suture placement device.

An extension mechanism, a pivot bar, is deployed from the housing, and positioned below the fascia. A channel is provided in the pivot bar, and extends from a receptor port on each side of the pivot bar, such that the receptor ports are located about 1 cm from opposed sides of the outside of the cannular housing. When the stylet guides are fully deployed, the distal end of each stylet guide engages a receptor port, thereby creating a suture path through the first stylet guide, into the first receptor port, through the channel, through the second receptor port, and through the second stylet guide. A stylet is then directed through the suture path to pull the first end of a suture through the path. After routing the suture, the stylet guides are retracted, leaving the suture routed through the fascia. The pivot bar has a suture release slot along the bottom of the pivot bar, and the suture is released through the suture release slot as the pivot bar is rotated back to a folded position. The suture placement device is withdrawn and the suture is tied.

FIG. 3A is a side perspective view of suture placement device **105** showing a body **510,** a pivot bar actuation slide **520,** a guide latch **530,** and a guide plunger **540.**

FIG. 3B is a side perspective view of the suture placement device with the extension mechanism pivot bar **550** deployed by sliding the pivot bar actuation slide **520.**

FIG. 3C is a side perspective view of the suture placement device with a first stylet guide **560** and a second stylet guide **570** deployed engaging receptor ports **554** and **555** on the pivot bar **550.** In this example, the first guide and second guide are deployed by pressing the guide plunger **540.**

FIG. 3D shows suture placement device **105** inserted into a tissue opening through the fascia layer **90** and fat layer **96.** The first guide **560** and the second guide **570** are deployed through the fascia layer **90.** A pivot bar **550** at the lower end of the suture placement device has been pivoted to move the pivot bar to an extended orientation below the fascia layer **91.** In this example, the extended pivot bar is perpendicular to the longitudinal axis of the suture placement device.

The first stylet guide **560** has created a suture path through the fascia layer **90** from an opening opening **501** in the cannular housing **510** to point **92** in the fascia above the first side of the pivot bar. The second stylet guide **570** has created a suture path through the fascia **90** from the opening **502** in the cannular housing **510** to point **93** in the fascia.

The pivot bar can engage the fascia **91** so that the surgeon can pull the suture placement device upwardly.

In this example, a clamp is not necessary to secure the suture placement device, and the suture placement device can be operated with a single hand as the deployed guides hold the device in place.

### creating the suture path

FIG. 4A shows a stylet **580** partially inserted into the first guide of the suture placement device. FIG. 4B shows the stylet **580** inserted into the extended first guide **560** and pivot bar channel **551** of the suture placement device. FIG. 4C shows further insertion of the stylet to create a suture path through the first guide **560,** through the pivot bar channel **551,** and through the second guide **570.** The first end **581** of the stylet has emerged from the suture placement device. As described below, a suture may be temporarily attached to the first end of the stylet, so that the suture is positioned in the suture path as the stylet is retracted back through the suture path. In other examples, the suture may be temporarily attached to the second end of the stylet, so that the suture is positioned in the suture path as the stylet is pulled through the suture path until the second end of the stylet emerges from the proximal end of second stylet guide **570.**

FIG. 4D shows the first end portion of a suture **590** attached to the second end **582** of stylet **580,** so that the stylet can pull the suture through the suture path. FIG. 4E shows the stylet **580** pulling the first end of a suture through the first guide **560** and channel **551** of the suture placement device. FIG. 4F shows the stylet pulling the end of a suture through the second guide **570.**

FIG. 4G shows the suture **590** positioned after the first guide and second guide are retracted, and the pivot bar is retracted. FIG. 44H shows the suture **590** remaining in place as the suture placement device **105** is partially removed.

In another example, the first end portion of a suture **590** is attached to the first end **581** of the stylet, and the stylet is then retracted until the first end of the stylet and the first end portion of a suture are pulled back through the proximal end of the first stylet guide.

### pivot bar

FIG. 5 is a side perspective view of a first half **550a** of pivot bar **550** showing half of the cross section of channel **551** and half of receptor port features **554** and **555.** The outside edge of the channel and the receptor ports have a suture release slot **553.** When this half and a second half of the pivot bar are assembled, the pivot bar provides a slotted channel **551** between the first receptor port **554** and the second receptor port **555.** The distal end of the first guide engages the first receptor port **554,** and the distal end of the second guide engages the second receptor port **555** to create a suture path through the guides, the receptor ports, and pivot bar channel. Suture is pulled through the suture path with a stylet, and is released through the suture release slot **553** as the pivot bar is retracted. In other examples, a pair of foldable wings may be used rather than a pivot bar.

### guide plunger

FIG. 6A is a top perspective view of the guide plunger **540.** FIG. 46B is a bottom perspective view of the plunger **540.** The plunger includes stylet ports **542** and **543** and stylet channels **547** and **548.** The distal ends of guides **560** and **570** are inserted into lower portion of the stylet channels **547** and **548** so that the guides and be extended or retracted with the plunger. In this example, a stylet is inserted into either of the stylet ports **542** or **543** and routed downward through the corresponding stylet channel into one of the guides, then through the pivot bar channel and back up through the other guide and back upward through the other stylet channel so that the stylet exits the other stylet port. In other examples as described below, a single entry port is provided on the proximal end of the device, and an exit port is provided through the side of the housing. In this example, suture release slots **545** and **546** extend from both stylet channels and stylet ports. In other examples, the suture release slots are not required and the suture remains in position as the device is removed. The guide plunger includes a latch recess **541** and a spring recess **549.**

FIG. 8 is a side view of a guide latch **530** for the suture placement device. The guide latch includes hook **582** for engaging the latch recess **541** of the guide plunger, and pivot hole **583,** and a latch grip 584.

### Guides

FIG. 7A is a side view of a first stylet guide **560** for the suture placement with the guide in a retracted position. FIG. 7B is a side view of the first guide **560** in an extended position when the guide plunger is depressed. FIG. 7C is a detailed side view of the proximal end of the first guide **560** showing a slot **562** on the guide. In other examples, slots on the guides are not required and the suture remains in position as the device is removed.

In the retracted position, the distal end portion **564** of the first guide **560** is confined within a directional channel within the body of the suture placement device. When the guide plunger is depressed, the distal portion **564** of the guide is pushed out of the body in a direction that will penetrate fascia and intercept the first receptor port **554** of the pivot bar. After placement of the suture, the first guide is retracted back into the body by the guide plunger so that the suture placement device may be removed. Suture remains in place as the first guide is retracted. In one example, the guide has a suture release slot to facilitate separation and release of the suture from the guide. In other examples, the suture release slot is not necessary, and the suture will remain in place as the guide is retracted.

Guides were initially constructed of nitinol, and subsequent testing has shown that stainless steel guides have worked effectively.

### body

FIG. 9A is a side perspective view of the outside of a first body section **510a** for the suture placement device. FIG. 9B is a side perspective view of the inside of a second body section **510a** for the suture placement device. FIG. 9C is a side view of the inside of the first body section of FIG. 9B. FIG. 9D is a bottom perspective view of the inside of the first body section of FIG. 9A.

Features for both body sections are similar so that internal features are created by mating the two body portions. The body sections include lower recess portions **511** to house the pivot bar. The pivot bar pivots with respect to pivot hole **518,** and is moved by actuation arms with respect to slot **519.**

The first guide is housed in a first guide channel **512** which includes first directional channel **513.** The second guide is housed in a second guide channel **514** which includes second directional channel **515.** In this example, the outside edges of the housing sections are recessed to provide suture release slots **517.** In other examples, suture release slots are not provided in along the body, and the suture remains in place as the suture placement device is removed.

### pivot bar actuation slides

FIG. 10A is a side perspective view of a first pivot bar actuation slide **520** which includes a shaft **522** extending from grip **521** to pin retainer **523.** As the pivot bar actuation slide is pressed downward with respect to the housing, the shaft **522** forces the retainer **523** to rotate the pivot bar wing to an open position. FIG. 10B is a side perspective view of a second pivot bar actuation slide **524** which includes a shaft **526** extending from grip **525** to pin retainer **527.** In one example, the first and second actuation slides are pinned together so that both slides may be operated by a single thumb of the user.

In one example, the pivot bar is spring loaded in a normally-extended position. The suture placement device is positioned in a trocar port with the pivot bar in a retracted position, aligned with the device. When the pivot bar reaches the cavity below the fascia, the spring-load will force the pivot bar to pivot to an open and extended position, and the actuation arms will move up and provide a visual indication that the suture placement device is in a proper position. The trocar port can then be removed.

### assembled device

FIGs. 11 and 12 are side perspective views of the suture placement device **105** with the pivot bar **550** in a retracted position. Suture release slot **517** is shown between body sections **510a** and **510b.** First guide exit port **501** is shown at the lower end of suture release slot **517.** The guide plunger **540** is in a raised position so that the first guide and second guide (not shown) are in retracted orientations inside the body. Pivot bar actuation slides **520** and **524** are shown in a retracted position.

FIG. 13 is a top perspective view of the assembled suture placement device **105** showing pivot bar receptor ports **554** and **555** and suture release slot **553.** The guide plunger **540** includes stylet ports **542** and **543.**

### TAP or other substance introduction

In this example, the pivot bar **550** serves as a fascial plane reference element and one or both stylet guides serves as an injection tube. A single guide plunger **540** may be used to set the depth of both stylet guides. The desired partial deployment depth of the distal end of the stylet guides may be set with a fixed mechanical stop or an adjustable mechanical stop on the guide plunger. FIG. 36 is a cross section view of a trocar port and trocar port site showing desired injection plane for TAP block.

FIG. 37A shows the guide plunger **540** partially depressed so that the stylet guide tips are positioned at a first distance from the fascial plane reference element. FIG. 37B shows the guide plunger **540** partially depressed so that the stylet guide tips are positioned at an optional second distance from the fascial plane reference element. FIG. 37C shows the guide plunger **540** fully depressed so that the stylet guide tips are fully deployed.

FIG. 38A shows the guide plunger **540** partially depressed according to a first color band or scale reading on the housing, so that the stylet guide tips are positioned at a first distance from the fascial plane reference element. FIG. 38B shows the guide plunger **540** partially depressed according to a second color band or scale reading on the housing, so that the stylet guide tips are positioned at an optional second distance from the fascial plane reference element. FIG. 38C shows the guide plunger **540** fully depressed so that the stylet guide tips are fully deployed.

In some cases, a single stylet guide can provide a single injection, and the injected substance will spread or migrate completely around a trocar port site. In other cases, a two stylet guides can be used to inject a substance on opposite sides of a trocar port site. An adapter may be provided to simultaneously introduce a substance or mixture to both stylet guides.

### Example- reusable metal suture placement device with proximal stylet ports

FIGs. 3-13 describe a reusable suture placement device that may be provided as a metal housing and metal pivot bar. In this example, the stylet ports **542** and **543** are located at the proximal end of the guide plunger.

### Example- reusable suture placement device with side stylet ports

In this example, the stylet ports are provided on the side of the housing in order to shorten the suture path and decrease the time required to route a stylet through the suture path.

### Example- reusable suture placement device with proximal and side stylet ports

In this example, an entry stylet port is provided on the proximal end of the device for convenient placement of the stylet, and an exit stylet port is provided on the side of the housing in order to shorten the suture path and decrease the time required to route a stylet through the suture path. In this example, the distance between the proximal stylet entry port to the first receptor port on the pivot bar (path segmentA-B-C in FIG. 2L) is longer than the distance between the first receptor port and the side stylet exit port (path segment C-C'-B'-A' in FIG. 2L). The shorter exit path length facilitates the use of a plunger in suture loading tools as described below.

### Example- disposable suture placement device with pivot bar

FIGs. 14-16 are a front view, rear view, and side perspective view of a plastic suture placement device **106** with a leur fitting **695** for a TAP procedure as described below. A leur lock may be removably or permanently attached to the proximal entry port to permit injection of an analgesic or other substance through the proximal stylet entry port and into a stylet guide.

FIG. 17 is an exploded side perspective view of the suture placement device **106** showing a plunger **640,** stylet guides **660** and **670,** an upper body **610,** lower body sections **616a** and **616b,** and pivot bar sections **650a** and **650b.**

FIGs. 18 and 19 are a front view and side perspective view of the upper body portion **610** of the plastic suture placement device of FIG. 14. The upper body portion includes recessed area **618** for a pivot bar knob, and recessed area **619** for a guide latch. The stylet exits through a stylet exit port **643** within slot **601.**

FIG. 20 is a top perspective view of the plunger **640** with stylet entry port **642.** In this example, a luer fitting **695** with an extension tube **696,** as shown in FIG. 21, may be inserted into the stylet entry port **642.** Depressing plunger **640** pushes connector tubes **685** and **686** and forces stylet guides **660** and **670** from a retracted to a deployed position. Depressing latch 630 permits plunger return spring **646** and latch spring **688** to push the plunger **640** and force stylet guides **660** and **670** from their deployed position back to a retracted orientation.

FIG. 22 is a front view of stylet guides **660.** FIG. 17 shows a first stylet guide **660** and a first connector tube **685.** A connector tube may be used where the bendable portion of the stylet guides are provided in a relatively expensive material such as nitinol. Testing has established that lower cost stainless steel guides are effective, so single piece stylet guides may be used in lieu of the two part stylet guide and connector tube design.

In this example, the distal end **664** of the first stylet guide and the distal end of the second stylet guide are tapered outward with respect to centerline of the housing. This outward tapering has provided consistent mating with the receptor ports on the pivot bar.

In this example, the lower body is provided in lower body sections **616a** and **616b.** FIG. 23 is a front view of the interior of lower body section **616b.** Stylet guides **660** and **670** are directed through stylet guide channels **647** and **648** so that the distal ends of the guides intercept the receptor ports on the pivot bar. FIG. 24 is a front perspective view of the exterior of lower body section **616a.**

In this example, the pivot bar is provided in sections **650a** and **650b.** FIG. 25 is a front perspective view of the pivot bar section **650b** showing portions of receptor ports **654** and **655,** suture release slot **653,** and pivot bar channel **651.** FIG. 26 is a front perspective view of the assembled pivot bar **650** with pivot bar rivet **652.** The pivot bar is rotated by moving the pivot bar actuation knob **625** which moves a single pivot bar actuation rod **620.** The pivot bar actuation rod pulls or pushes the pivot bar rivet **652,** which causes the pivot bar to rotate with respect to pivot bar roll pins **657a** and **657b.** In this example, a pivot bar actuation spring **626** biases the pivot bar to an extended position.

### Example- suture placement device with disposable or replaceable guides

In this example, a metal or other reusable suture placement device body is hinged to provide access to the stylet guides, so that the stylet guides may be removed and replaced. In other examples, the pivot bar may be removed and replaced.

### Example- suture placement device used as obturator

In this example, a suture placement device with a pivot bar is adapted to serve as an obturator and a closure device. FIG. 27A shows a suture placement device that is configured to be used as an obturator **701.** In this example, a removable head **710** is placed over the suture placement device and trocar to secure the device as it is used to pierce the fascia during initial placement of the trocar port. The suture placement device/obturator is placed inside the trocar port (not shown) during this operation. The leading edge **721** of the pivot bar on the suture placement device is sharp to penetrate the fascia during trocar installation. FIG. 27B shows the suture placement device/obturator of FIG.27A with the head **710** removed so that the device is configured to be used as a suture placement device. FIG. 27C shows the pivot bar **720** deployed when the device of FIG. 27B is inserted into a trocar port (not shown). The pivot bar has a sharp first tip **721** and a rounded second tip **722.** In one example, the sharp first tip **721** is rotated 180 degrees after its use as an obturator tip so that the rounded second tip **722** becomes the leading tip when the suture placement device is re-inserted into a trocar port to serve as a closure device.

### Example- suture placement device for 10mm trocars

In this example, the suture placement device is sized to fit inside a 10mm or larger diameter trocar, so that the trocar can be removed over the device.

### Example- suture placement device for 8mm trocars

In this example, a suture placement device designed for insertion into a 10mm trocar is inserted into an 8mm trocar port site. Referring to FIG 28A, the proximal end of a stylet **580** is inserted into the trocar. The trocar is then removed over the stylet. After removing the trocar, the distal end of the stylet **580** is inserted into the distal end of stylet guide **560** of a suture placement device **106** without deploying the stylet guides as shown in FIG. 28B. The stylet is pushed through the stylet guide **560** until the distal end of the stylet is pushed out of the stylet entry port. The stylet is then grasped and used as a guide wire as the suture placement device is inserted into the trocar port site. Referring to FIG. 28C, the pivot bar **550** is deployed and a slight upward pressure is applied to the suture placement device as the stylet is removed until the proximal end of the stylet **580** is drawn into the stylet guide 560. The stylet guides are then deployed and the stylet is fed through the device as described above.

### DESCRIPTION OF EMBODIMENT- Suture loading devices

In the examples described above, a stylet is used to route a suture through the suture path. In various device testing, a trocar port site can be closed in about 60 seconds or less, with about 10-30 seconds required to route a stylet. Several example suture loading devices have been developed in order to reduce the required closure time by reducing stylet load time.

FIGs 29A-29E are cross section schematics illustrating a suture loading device **750** to route a suture through a suture path.

FIGs. 29A-29B illustrate a suture loading technique using a suture loading device **750.** In this example, a flexible leading shaft portion **762** is inserted into a stylet guide entry port. In a first step, as illustrated by FIG. 29A, the flexible leading shaft portion **762** is contained within a stylet guide of the suture placement device and extends from below point A at the proximal end of the suture placement device to point Cat the first receptor port on the pivot bar. This positioning of the flexible leading shaft portion **762** within the stylet guide protects the flexible leading shaft portion to minimize kinking.

FIG. 29B illustrates a more rigid push shaft section **764** of the suture loading device **750** pushed downward with handle **780** so that the distal end **765** of the push shaft section **764** is at or near point C. In this example, the push shaft section is flexible enough to be pushed through the guide from point B to point C, but not flexible enough to make the bend in the suture path at point C. In this example, the path A-B-C is longer than the path C-C'-B'-A' so that pushing the push shaft section to point C forces the flexible leading shaft portion **762** through path C-C'-B'-A' thereby causing the distal end **763** of the flexible leading shaft portion **762** to emerge from the suture placement device at point A'. As described below, the suture may either be grasped by the suture loading device and pulled back through the pathA'-B'-C'-C-B-A as the suture loading device **750** is withdrawn, or the suture may have been carried through the suture path so that the suture can be released and held while the suture loading device is withdrawn.

In one example, suture capture is performed on the flexible leading shaft portion **762** before the suture loading device is inserted into the suture placement device. After the flexible leading shaft portion **762** emerges from the suture placement device at point A', the the suture may be removed. The suture loading device **750** is then withdrawn while the suture end is held at point A'.

In another example, suture capture is performed after the suture loading device is inserted into the suture placement device and the flexible leading shaft portion **762** emerges from the suture placement device at point A' as illustrated by FIG. 29B.

In FIG. 29C, the suture loading device is partially withdrawn from the suture placement device so that the first end of the suture is between points A' and B'.

In FIG. 29D, the suture loading device is almost withdrawn from the suture placement device so that the first end of the suture is between points A and B.

In FIG. 29E, the suture loading device is fully withdrawn from the suture placement device so that the first end of the suture has been pulled out of the suture placement device at point A; and the handle has been depressed to release the suture.

### Example- Suture loading device

FIG. 30A is a front perspective view of a suture loading device **751** that is used to pull a suture **70** though a suture path. The suture loading device **751** has a two-part shaft **760** comprising a flexible leading shaft portion **762** and a push shaft section **764.**

FIG. 30B is a detailed cross section view of a handle assembly **770** with a handle **790** and a collar **782.** The collar **782** has a handle opening **774,** an interior chamber **775,** and a shaft opening **776.** In this example, the collar includes a circumferential finger recess **781** configured so that the collar can be grasped between two fingers as the handle **782** is depressed with the palm of the hand or thumb.

FIG.30C is a detailed view of the distal end **763** of suture loading device **751** with the device in its normally-closed orientation.

FIG. 30D is a detailed cross section view of a handle assembly **770** with the handle **790** in a suture capture/release orientation.

FIG. 30E is a detailed view of the distal end of suture loading device **751** with the device in a suture capture orientation. In this example, two wires **752** and **754** are routed from the handle and through the two part shaft. The proximal end of the wires are attached to distal end **792** of the handle, so that as the handle is depressed toward the collar, the distal end of the two wires are extended past the distal end **763** of the flexible leading shaft portion **762.** The distal ends of the two wires are joined with a ball **755** that has a diameter greater than the inside diameter of the flexible leading shaft portion **762.** When the wires are retracted, the ball **755** serves to assist in pushing the flexible leading shaft portion through the suture path. When the distal end of the wires and ball are extended past the distal end of the distal end **763** of the flexible leading shaft portion **762,** a portion of a suture may be placed over the exposed wires so that when the handle is released, the ball **755** will be retracted to the distal end **763** of the flexible leading shaft portion and will secure the suture between the ball **755** and the distal end **763** of the flexible leading shaft portion **762.** The suture may be released by moving the handle back to the capture/release orientation.

In one example, the suture may be captured on the flexible leading shaft portion **762** before the suture loading device is inserted into the suture placement device. After the flexible leading shaft portion **762** emerges from the suture placement device at point A', the handle is depressed so that the suture may be removed. The suture loading device **751** is then withdrawn while the suture end is held.

In another example, suture capture is performed after the suture loading device is inserted into the suture placement device and the flexible leading shaft portion **762** emerges from the suture placement device at point A'. The handle is depressed so that the suture may be attached, the suture loading device is withdrawn from the suture placement device, and the handle is depressed again so that the suture may be removed.

### Stylet ball tip

Stylet loading has been improved by modifying the lead tip of a stylet by attaching a ball such as shown in FIG. 30C. The ball appears to minimize stylet hangups in the suture path.

### Example- Suture loading device

FIG. 31A is a front perspective view of another suture loading device **756.** The suture loading device **756** has a handle assembly **771,** and a two-part shaft **760** comprising a flexible leading shaft portion **762** and a push shaft section **764.** The flexible leading shaft portion **762** has a gap **753** that is expanded or contracted by operating the handle.

FIG. 31B is a detailed view of a handle assembly **771.**

FIG. 31C is a detailed view of the distal end portion of suture loading device **756** with gap **753** opened to capture a suture between wires **752** and **754** which are forced apart as the handle is operated. The suture is also released by opening gap **753.**

### Stylet plunger

In another example, a portion of a stylet is loaded into the suture placement device, and a plunger **791** such as illustrated in FIG. 32 is used to push the leading portion of the stylet through the suture path. The plunger in FIG. 32 comprises a handle **793,** and a relatively rigid shaft **794.**

While exemplary embodiments of the invention have been described, it should be apparent that modifications and variations thereto are possible, which may fall within the scope of the invention as defined by the appended claims. With respect to the above description then, it is to be realized that the optimum relationships for the components of the invention, including variations in order, form, content, function and manner of operation, are deemed readily apparent and obvious to one skilled in the art, and all equivalent relationships to those illustrated in the drawings and described in the specification may be encompassed by the present invention as defined by the appended claims. The above description and drawings are illustrative of modifications that can be made without departing from the present invention, the scope of which is to be limited only by the following claims. Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications and equivalents may fall within the scope of the invention as claimed.

## Claims

1. A suture placement device for the placement of a suture after the removal of an endoscopic trocar port, the suture placement device comprising:
a cannular housing (510) configured to be insertable into a trocar port (85) of a determined width, the cannular housing comprising:
a proximal portion and a distal portion, and having a longitudinal axis extending from the proximal portion to the distal portion;
an extension mechanism (550) movably coupled to the distal portion of the cannular housing, the extension mechanism comprising:
a first segment with a first terminus and a first receptor in proximity to the first terminus, and
a second segment with a second terminus and a second receptor in proximity to the second terminus,
the extension mechanism being movable between
a folded position, with the first segment and second segment being oriented for passage through the trocar port, and
an extended position, with a lateral axis extending from the first terminus to the second terminus and being substantially orthogonal to the longitudinal axis,
and
a first stylet guide (560) and a second stylet guide (570), each stylet guide comprising:
a proximal end,
a distal end, and
a stylet guide suture path segment between the proximal end and the distal end,
each stylet guide being movable between
a retracted position such that the stylet guide is oriented for passage through the trocar port, and
a deployed position where a portion of the stylet guide extends from the housing to one of the first and second receptors of the extension mechanism,
**characterised in that** the first receptor is a first receptor port (554), **in that** the second receptor is a second receptor port (555), **in that** the first segment and second segment provide a distal suture passage (551) extending from the first receptor port to the second receptor port, and **in that** the distal suture passage includes a suture release slot (553).

2. The suture placement device of claim 1 wherein the extension mechanism is a pivot bar (550).

3. The suture placement device of claim 1 further comprising
an extension mechanism deployment mechanism (540) comprising
a thumb latch (530) or finger latch;
a return spring; and
a lock.

4. The suture placement device of claim 1 wherein the cannular housing further comprises
a first stylet guide alignment channel (647); and
a second stylet guide alignment channel (648).

5. The suture placement device of claim 1 further comprising
a stylet guide deployment mechanism comprising
a thumb-driven actuator (640),
a first lock configured to hold the first and second stylet guides in a fully deployed position, and a return spring (688).

6. The suture placement device of claim 5 wherein the stylet guide deployment mechanism further comprises
a second lock configured to hold the first stylet guide in a partially deployed position.

7. The suture placement device of claim 1 further comprising
a stylet suture loading element (756).

8. The suture placement device of claim 1 wherein
the first receptor port of the extension mechanism is positioned approximately one centimeter from the cannular housing; and
the second receptor port of the extension mechanism is positioned approximately one centimeter from the cannular housing and opposed to the first receptor port.

9. The suture placement device of claim 1 wherein
the first stylet guide is movable between
a retracted position,
a partially deployed position where the distal end of the first stylet guide is spaced apart from the first receptor port by a desired distance in the range of about 0.1 to 1.0 cm, and
a fully deployed position where the distal end of the first stylet guide is extended to the first receptor port of the extension mechanism; and
the proximal end of the first stylet guide is configured to be in fluid communication with a syringe, needle, or injection tube.

## Patentansprüche

1. Nahtplatzierungsvorrichtung zum Platzieren einer Naht nach dem Entfernen eines endoskopischen Trokar-Ports, die Nahtplatzierungsvorrichtung umfassend:
ein Kanülengehäuse (510), das so konfiguriert ist, dass es in einen Trokar-Port (85) einer bestimmten Breite eingeführt werden kann, das Kanülengehäuse umfassend:
einen proximalen Abschnitt und einen distalen Abschnitt, und aufweisend eine Längsachse, die sich ausgehend von dem proximalen Abschnitt zu dem distalen Abschnitt erstreckt;
einen Verlängerungsmechanismus (550), der bewegbar mit dem distalen Abschnitt des Kanülengehäuse gekoppelt ist, der Verlängerungsmechanismus umfassend:
ein erstes Segment mit einer ersten Endpunkt und einem ersten Rezeptor in der Nähe des ersten Endpunkts, und
ein zweites Segment mit einem zweiten Endpunkt und einem zweiter Rezeptor in der Nähe der zweiten Endpunkts,
wobei der Verlängerungsmechanismus bewegbar ist zwischen
einer gefalteten Position, in welcher das erste Segment und das zweite Segment für die Passage durch den Trokar-Port hindurch ausgerichtet sind, und
einer verlängerten Position, in welcher sich eine laterale Achse ausgehend von dem ersten Endpunkt zu dem zweiten Endpunkt erstreckt und im Wesentlichen senkrecht zu der Längsachse ist,
und
eine erste Mandrin-Führung (560) und eine zweite Mandrin-Führung (570), jede Mandrin-Führung umfassend:
ein proximales Ende,
ein distales Ende, und
ein Mandrin-Führungs-Nahtstreckensegment zwischen dem proximalen Ende und dem distalen Ende,
wobei jede Mandrin-Führung bewegbar ist zwischen
einer eingefahrenen Position, so dass die Mandrin-Führung für die Passage durch den Trokar-Port hindurch ausgerichtet ist, und
einer ausgefahrenen Position, in welcher sich ein Abschnitt der Mandrin-Führung ausgehend von dem Gehäuse zu einem der ersten und zweiten Rezeptoren des Verlängerungsmechanismus erstreckt,
**dadurch gekennzeichnet, dass** der erste Rezeptor ein erster Rezeptor-Port (554) ist, dass der zweite Rezeptor ein zweiter Rezeptor-Port (555) ist, dass das erste Segment und das zweite Segment eine distale Nahtpassage (551) bereitstellen,
die sich ausgehend von dem ersten Rezeptor-Port zu dem zweiten Rezeptor-Port erstreckt, und dass die distale Nahtpassage einen Nahtfreigabeschlitz (553) enthält.

2. Nahtplatzierungsvorrichtung nach Anspruch 1, wobei der Verlängerungsmechanismus eine Gelenkstange (550) ist.

3. Nahtplatzierungsvorrichtung nach Anspruch 1, des Weiteren umfassend einen Verlängerungsmechanismus-Ausfahrmechanismus (540), umfassend
ein Daumenarretierteil (530) oder Fingerarretierteil;
eine Rückholfeder; und
eine Sperre.

4. Nahtplatzierungsvorrichtung nach Anspruch 1, wobei das Kanülengehäuse des Weiteren umfasst
einen ersten Mandrin-Führungs-Ausrichtungskanal (647); und
einen zweiten Mandrin-Führungs-Ausrichtungskanal (648).

5. Nahtplatzierungsvorrichtung nach Anspruch 1, des Weiteren umfassend
einen Mandrin-Führungs-Ausfahrmechanismus, umfassend
ein daumenbetätigtes Stellglied (640),
eine erste Sperre, die dazu konfiguriert ist, die ersten und zweiten Mandrin-Führungen im einer vollständig ausgefahrenen Position zu halten, und eine Rückholfeder (688).

6. Nahtplatzierungsvorrichtung nach Anspruch 5, wobei der Mandrin-Führungs-Ausfahrmechanismus des Weiteren umfasst
eine zweite Sperre, die dazu konfiguriert ist, die erste Mandrin-Führung in einer teilweise ausgefahrenen Position zu halten.

7. Nahtplatzierungsvorrichtung nach Anspruch 1, des Weiteren umfassend ein Mandrin-Naht-Ladeelement (756).

8. Nahtplatzierungsvorrichtung nach Anspruch 1, wobei
der erste Rezeptor-Port des Verlängerungsmechanismus ungefähr einen Zentimeter von dem Kanülengehäuse entfernt positioniert ist; und
der zweite Rezeptor-Port des Verlängerungsmechanismus ungefähr einen Zentimeter von dem Kanülengehäuse entfernt und gegenüber dem ersten Rezeptor-Port positioniert ist.

9. Nahtplatzierungsvorrichtung nach Anspruch 1, wobei
die erste Mandrin-Führung bewegbar ist zwischen
einer eingefahrenen Position,
einer teilweise ausgefahrenen Position, in welcher das distale Ende der ersten Mandrin-Führung zu dem ersten Rezeptor-Port um eine gewünschte Entfernung im Bereich von ungefähr 0,1 bis 1,0 cm beabstandet ist, und einer vollständig ausgefahrenen Position, in welcher das distale Ende der ersten Mandrin-Führung bis zum ersten Rezeptor-Port des Verlängerungsmechanismus verlängert ist; und
wobei das proximale Ende der ersten Mandrin-Führung dazu konfiguriert ist, in Fluidkommunikation mit einer Spritze, einer Nadel oder einem Injektionsrohr zu stehen.

## Revendications

1. Un dispositif de placement de suture pour le placement d'une suture après le retrait d'un orifice de trocart endoscopique, le dispositif de placement de suture comprenant :
un logement de canule (510) configuré pour pouvoir être inséré dans un orifice de trocart (85) d'une largeur déterminée, le logement de canule comprenant :
une partie proximale et une partie distale, et ayant un axe
longitudinal s'étendant de la partie proximale à la partie distale ;
un mécanisme d'extension (550) couplé de façon mobile à la partie distale du logement de canule, le mécanisme d'extension comprenant :
un premier segment avec une première fin et un premier récepteur à proximité de la première fin, et
un deuxième segment avec une deuxième fin et un deuxième récepteur à proximité de la deuxième fin, le mécanisme d'extension étant mobile entre
une position pliée, le premier segment et le deuxième segment étant orientés pour un passage à travers l'orifice de trocart, et
une position étendue, un axe latéral s'étendant de la première fin à la deuxième fin et étant substantiellement orthogonal à l'axe longitudinal,
et
un premier guide stylet (560) et un deuxième guide stylet (570), chaque guide stylet comprenant :
une extrémité proximale,
une extrémité distale, et
un segment de trajet de suture de guide stylet entre l'extrémité
proximale et l'extrémité distale,
chaque guide stylet étant mobile entre
une position rétractée de telle sorte que le guide stylet soit orienté pour un passage à travers l'orifice de trocart, et
une position déployée où une partie du guide stylet s'étend du logement à l'un des premier et deuxième récepteurs du mécanisme d'extension,
**caractérisé en ce que** le premier récepteur est un premier orifice récepteur (554), **en ce que** le deuxième récepteur est un deuxième orifice récepteur (555), **en ce que** le premier segment et le deuxième segment fournissent un passage de suture distal (551) s'étendant du premier orifice récepteur au deuxième orifice récepteur, et **en ce que** le passage de suture distal inclut une fente de libération de suture (553).

2. Le dispositif de placement de suture de la revendication 1 dans lequel le mécanisme d'extension est une barre de pivot (550).

3. Le dispositif de placement de suture de la revendication 1 comprenant en outre
un mécanisme de déploiement de mécanisme d'extension (540) comprenant
une clenche (530) ou un loquet à doigt ;
un ressort de rappel ; et
un verrou.

4. Le dispositif de placement de suture de la revendication 1 dans lequel le logement de canule comprend en outre
un premier canal d'alignement de guide stylet (647) ; et
un deuxième canal d'alignement de guide stylet (648).

5. Le dispositif de placement de suture de la revendication 1 comprenant en outre
un mécanisme de déploiement de guide stylet comprenant
un actionneur entraîné par un pouce (640),
un premier verrou configuré pour maintenir les premier et deuxième guides stylet dans une position complètement déployée, et un ressort de rappel (688).

6. Le dispositif de placement de suture de la revendication 5 dans lequel le mécanisme de déploiement de guide stylet comprend en outre un deuxième verrou configuré pour maintenir le premier guide stylet dans une position partiellement déployée.

7. Le dispositif de placement de suture de la revendication 1 comprenant en outre
un élément de chargement de suture par stylet (756).

8. Le dispositif de placement de suture de la revendication 1 dans lequel
le premier orifice récepteur du mécanisme d'extension est positionné à approximativement un centimètre du logement de canule ; et
le deuxième orifice récepteur du mécanisme d'extension est positionné à approximativement un centimètre du logement de canule et opposé au premier orifice récepteur.

9. Le dispositif de placement de suture de la revendication 1 dans lequel
le premier guide stylet est mobile entre
une position rétractée,
une position partiellement déployée où l'extrémité distale du premier guide stylet est espacée du premier orifice récepteur d'une distance souhaitée dans la plage allant d'environ 0,1 à 1,0 cm, et
une position complètement déployée où l'extrémité distale du premier guide stylet s'étend jusqu'au premier orifice récepteur du mécanisme d'extension ; et
l'extrémité proximale du premier guide stylet est configurée pour être en communication fluidique avec une seringue, une aiguille, ou un tube d'injection.
